# EUROPÄISCHE PATENTSCHRIFT

(11) **EP 0 332 968 B1**
(45) Veröffentlichungstag und Bekanntmachung des Hinweises auf die Patenterteilung: **02.06.1993**
(21) Anmeldenummer: 89103871.3
(22) Anmeldetag: 06.03.1989
(51) Int. Cl.: C07D 417/12, C07D 209/90, C07D 401/12, C07D 403/12, A61K 31/40, A61K 31/425

(54) **1,3,4,5-Tetrahydrobenz[c,d]indole**
1,3,4,5-Tetrahydrobenz[c,d]indoles
Tétrahydro-1,3,4,5 benzo[c,d]indoles

(30) Priorität: 18.03.1988 DE 3809155
(43) Veröffentlichungstag der Anmeldung: 20.09.1989
(73) Patentinhaber: BAYER AG, 51368 Leverkusen (DE)
(72) Erfinder: Junge, Bodo, Dr., D-5600 Wuppertal 23 (DE); Richter, Bernd, Dr., D-5060 Bergisch Gladbach (DE); Glaser, Thomas, Dr., D-5064 Roesrath (DE); Traber, Jörg, Dr., D-5204 Lohmar (DE)

(56) Entgegenhaltungen:
- EP-A- 153 083
- EP-A- 162 695
- EP-A- 0 207 695
- EP-B- 0 029 581
- DE-A- 3 346 573
- FR-A- 2 471 373
- US-A- 4 110 339

## Beschreibung

Die Erfindung betrifft substituierte 1,3,4,5-Tetrahydrobenz[c,d]indole, Verfahren zu ihrer Herstellung und ihre Verwendung in Arzneimitteln.

Aus der DE 33 46 573, der EP 153 083 und der EP 162 695 ist bereits bekannt, daß 6-substituierte 1,3,4,5-Tetrahydrobenz[c,d]indol-4-amine eine hohe Affinität zu Serotoninrezeptoren des 5-HT₁-Typs besitzen und zur Behandlung von Krankheiten verwendet werden können.

Es wurden nun neue 6-substituierte 4-Amino-1,3,4,5-tetrahydrobenz[c,d]indole der allgemeinen Formel (I), 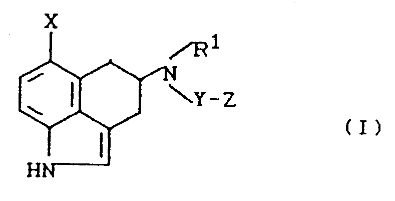
in welcher
- R¹: für H, C₁-C₄-Alkyl oder Benzyl steht,
- X: für H, OCH₃, OH, SCH₃, F, Cl, Br, CN oder CONH₂ steht,
- Y: für eine geradkettige oder verzweigte, gesättige oder ungesättigte Alkylekette mit bis zu 6 Kohlenstoffatomen steht,
und
- Z: für Cyano steht, oder für eine Gruppe der Formel 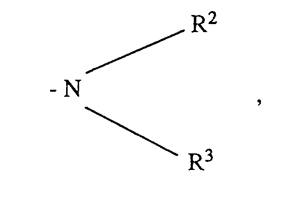 -SO₂NR⁷R⁸ oder CONR⁷R⁸ steht,
worin
- R⁷ und R⁸: gleich oder verschieden sind und
für Wasserstoff, C₁-C₆-Alkyl, Phenyl, Benzyl oder Phenethyl stehen,
- R² und R³: gleich oder verschieden sind und
für Wasserstoff oder C₁-C₆-Alkyl, Phenyl oder Benzyl stehen, wobei die Phenylreste durch Fluor, Chlor, Brom, C₁-C₆-Alkyl, C₁-C₆-Alkoxy oder Trifluormethyl substituiert sein können, oder
für eine Gruppe der Formel
COR⁹ oder -SO₂R¹⁰ stehen,
worin
- R⁹: Wasserstoff oder eine Gruppe NHR¹¹ bedeutet, oder C₁-C₆-Alkyl oder C₁-C₆-Alkoxy bedeutet, oder gegebenenfalls durch C₁-C₆-Alkyl, C₁-C₆-Alkoxy, Fluor, Chlor, Brom, Trifluormethyl, Dimethylamino oder Diethylamino substituiertes Phenyl, Benzyl, Benzyloxy, Thienyl, Furyl, Pyridyl, Pyrimidyl, Chinolyl, Isochinolyl, Benzothiazolyl, Benzoxazolyl, Thiozolyl, Oxazolyl, Isoxazolyl oder Isothiozolyl bedeutet,
- R¹⁰: gegebenenfalls durch Cyano, Fluor, Chlor, Brom, Trifluormethyl oder C₁-C₆-Alkoxycarbonyl substituiertes C₁-C₆-Alkyl bedeutet, oder gegebenenfalls durch C₁-C₄-Alkyl, C₁-C₆-Alkoxy, Fluor, Chlor, Brom, Trifluormethyl, Dimethylamino oder Dimethylamino substituiertes Phenyl, Naphthyl, Benzyl, Thienyl, Furyl, Pyrimidyl, Pyridyl, Chinolyl, Isochinolyl, Benzothiazolyl, Benzoxazolyl, Thiazolyl, Oxazolyl, Isoxazolyl oder Isothiazolyl bedeutet, oder
eine Gruppe -NR⁷R⁸ bedeutet,
wobei
R⁷ und R⁸ die oben angegebene Bedeutung haben,
und
- R¹¹: gegebenenfalls durch Cyano, Fluor, Chlor oder Brom substituiertes C₁-C₆-Alkyl bedeutet, oder gegebenenfalls durch C₁-C₆-Alkyl, C₁-C₆-Alkoxy, Fluor, Chlor, Brom, Trifluormethyl, Dimethylamino oder Diethylamino substituiertes Phenyl, Benzyl, Thienyl, Furyl, Pyridyl, Pyrimidyl, Chinolyl, Isochinolyl, Benzothiazolyl, Benzoxazolyl, Thiazolyl, Oxazolyl, Isoxazolyl oder Isothiazolyl bedeutet,
oder
- R² und R³: zusammen mit dem Stickstoffatom einen Heterocyclus der Reihe 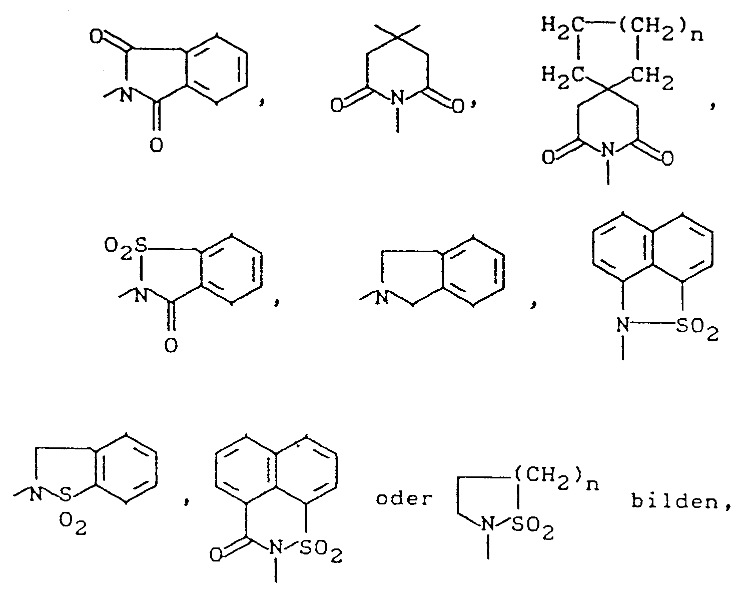 worin
- n: eine Zahl 1 oder 2 bedeutet
und deren Salze,
gefunden.

Die erfindungsgemäßen Stoffe haben mehrere asymmetrische Kohlenstoffatome und können daher in verschiedenen stereochemischen Formen existieren. Darüberhinaus können Verbindungen mit einer Sulfoxidgruppe ebenfalls in unterschiedlichen stereochemischen Formen existieren. Sowohl die einzelnen Isomeren, als auch deren Mischungen sind Gegenstand der Erfindung. Beispielsweise seien folgende isomeren Formen der basisch substituierten 1,3,4,5-Tetrahydrobenz[c,d]indole genannt

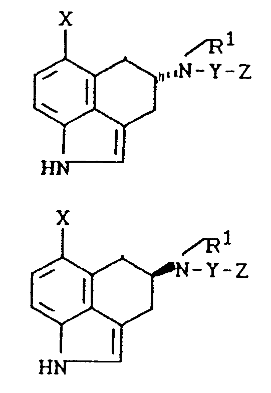

Die erfindungsgemäßen basisch substituierten 1,3,4,5-Tetrahydrobenz[c,d]indole können auch in Form ihrer Salze vorliegen. Im allgemeinen seien hier Salze mit anorganischen oder organischen Säuren genannt.

Im Rahmen der vorliegenden Erfindung werden physiologisch unbedenkliche Salze bevorzugt. Physiologisch unbedenkliche Salze der basisch substituierten 1,3,4,5-Tetrahydrobenz[c,d]indole können Salze der erfindungsgemäßen Stoffe mit Mineralsäuren, Carbonsäuren oder Sulfonsäuren sein. Besonders bevorzugt sind beispielsweise Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Oxalsäure, Malonsäure, Bernsteinsäure, Maleinsäure, Fumarsäure, Äpfelsäure, Weinsäure, Zitronensäure oder Benzoesäure.

Die erfindungsgemäßen Stoffe zeigen überraschenderweise eine vorteilhafte Wirkung auf das Zentralnervensystem und können zur therapeutischen Behandlung von Menschen und Tieren verwendet werden. Gegenüber den bereits bekannten strukturverwandten Verbindungen zeichnen sie sich vor allem durch eine bessere Verträglichkeit aus.

Bevorzugt werden Verbindungen der Formel (I),
in welcher
- R¹: für H, CH₃, Ethyl, n-Propyl oder Benzyl steht,
- X: für H, OCH₃, OH, SCH₃, F, Cl, CN, CONH₂ steht,
- Y: für eine geradkettige Alkylenkette mit 2 bis 4 Kohlenstoffatomen steht
und
- Z: für Cyano steht, oder für eine Gruppe der Formel 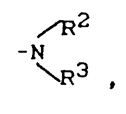 -SO₂NR⁷R⁸, -CONR⁷R⁸ steht,
wobei
- R⁷ und R⁸: gleich oder verschieden sind und
für Wasserstoff, Methyl, Ethyl, Propyl, Isopropyl, Butyl, Isobutyl oder tert.Butyl stehen,
- R² und R³: gleich oder verschieden sind, und
für Wasserstoff, Methyl, Ethyl, Propyl, Isopropyl, Butyl, Isobutyl, tert.Butyl oder für gegebenenfalls durch Fluor, Chlor, Methyl
oder Methoxy subsituiertes Phenyl, oder
für eine Gruppe -COR⁹ oder -SO₂R¹⁰ stehen,
worin
- R⁹: Wasserstoff oder eine Gruppe NHR¹¹ bedeutet, oder Methyl, Ethyl, Propyl, Isopropyl, Methoxy, Ethoxy, Propoxy, Isopropoxy, oder gegebenenfalls durch Methyl, Methoxy, Fluor oder Chlor substituiertes Phenyl, Benzyl, Benzyloxy, Thienyl, Furyl, Pyridyl, Pyrimidyl, Chinolyl oder Isochinolyl bedeutet,
- R¹⁰: gegebenenfalls durch Fluor, Chlor, Methoxycarbonyl, Ethoxycarbonyl, Propoxycarbonyl, Isopropoxycarbonyl, Butoxycarbonyl oder Isobutoxycarbonyl substituiertes Methyl, Ethyl, Propyl, Isopropyl, Butyl oder Isobutyl bedeutet, oder gegebenenfalls durch Methyl, Ethyl, Propyl, Isopropyl, Methoxy, Fluor oder Chlor substituiertes Phenyl, Naphthyl, Benzyl, Thienyl, Furyl, Pyridyl, Pyrimidyl, Chinolyl oder Isochinolyl bedeutet, oder
eine Gruppe NR⁷R⁸ bedeutet,
wobei
R⁷ und R⁸ die oben angegebene Bedeutung haben,
und
- R¹¹: gegebenenfalls durch Fluor oder Chlor substituiertes Methyl, Ethyl, Propyl, Isopropyl, Butyl, Isobutyl, Pentyl, Isopentyl, Hexyl oder Isohexyl bedeutet, oder Phenyl oder Benzyl bedeutet, das durch Fluor, Chlor, Methyl oder Methoxy substituiert sein kann,
oder
- R² und R³: gemeinsam mit dem Stickstoffatom einen Heterocyclus der Reihe 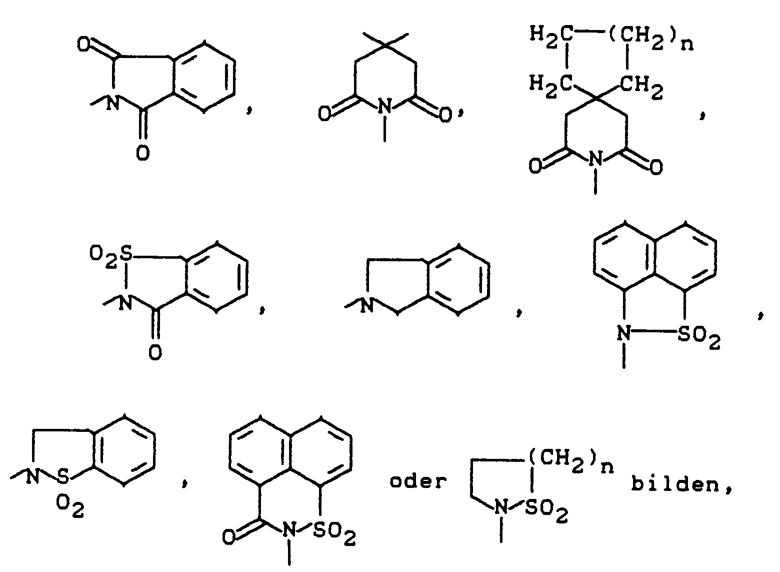 worin
- n: eine Zahl 1 oder 2 bedeutet
und deren Salze.

Als erfindungsgemäße Verbindungen seien beispielhaft genannt:
6-Methoxy-4-[4-(N-1,2-benzisothiazol-3(2H)-on-1,1-dioxid-yl)]-butylamino-1,3,4,5-tetrahydrobenz[c,d]indol Hydrochlorid,
4-[4-(N-1,2-benzisothiazol-3(2H)-on-1,1-dioxid-yl)]-butylamino-1,3,4,5-tetrahydrobenz[c,d]-indol,
6-Methoxy-4-[N-propyl-N-(ethyloxycarbonylaminoethyl)]-amino-1,3,4,5-tetrahydrobenz[c,d]-indol,
6-Methoxy-4-[N-propyl-N-(methylsulfonylamidoethyl)]-amino-1,3,4,5-tetrahydrobenz[c,d]-indol,
6-Methoxy-4-[N-propyl-N-(butylsulfonylamidoethyl)]-amino-1,3,4,5-tetrahydrobenz[c,d]-indol,
6-Methoxy-4-[N-propyl-N-(tosylamidoethyl)]-amino-1,3,4,5-tetrahydrobenz[c,d]-indol Hydrochlorid,
6-Methoxy-4-[N-propyl-N-(2-naphthylsulfonylamidoethyl)]-amino-1,3,4,5-tetrahydrobenz[c,d]-indol-Hydrochlorid
6-Methoxy-4-[N-propyl-N-(1-phenylureidoethyl)]-amino-1,3,4,5-tetrahydrobenz[c,d]-indol,
6-Methoxy-4-(dimethylsulfamoylethyl)-amino-1,3,4,5-tetrahydrobenz[c,d]-indol,
6-Methoxy-4-[N-propyl-N-(dimethylsulfamoylethyl)]-amino-1,3,4,5-tetrahydrobenz[c,d]-indol,
6-Methoxy-4-(methylsulfonylamidopropyl)-amino-1,3,4,5-tetrahydrobenz[c,d]-indol Hydrochlorid,
6-Methoxy-4-[N-benzyl-N-(methylsulfonylamidopropyl)]-amino-1,3,4,5-tetrahydrobenz[c,d]-indol,
6-Methoxy-4-(4-fluorphenylsulfonylamidopropyl)-amino-1,3,4,5-tetrahydrobenz[c,d]-indol Hydrochlorid,
6-Methoxy-4-[N-benzyl-N-(4-fluorphenylsulfonylamidopropyl)]-amino-1,3,4,5-tetrahydrobenz[c,d]-indol.

Weiterhin wurde gefunden, daß man Verbindungen der Formel (I) erhält,
indem man
Ketone der Formel (II) 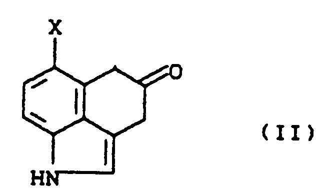
in der X die oben angegebene Bedeutung besitzt,
mit Aminen der Formel (III), (IV) oder (V) 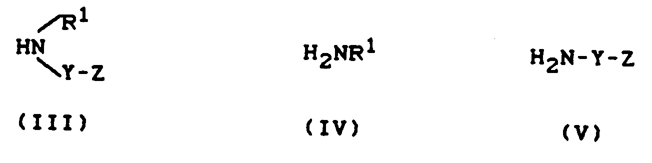
in denen R¹, Y und Z die oben angegebene Bedeutung haben, reduktiv aminiert [A].

Desweiteren erhält man Verbindungen der allgemeinen Formel (I)
wenn man
Verbindungen der allgemeinen Formel (VI) 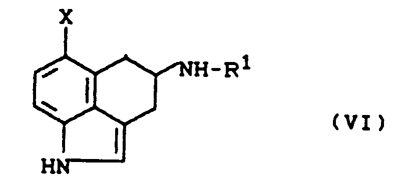
in der R¹ und X die oben angegebene Bedeutung haben,
mit Alkylierungsmitteln der Formel (VII)

L-Y-Z (VII)

in der Y und Z die oben angegebene Bedeutung haben, und L eine in Alkylierungsmitteln übliche "leaving group" wie Cl, Br, J, OTs, OMs oder OSO₂CF₃ bedeutet, umsetzt [B],
oder mit Aldehyden der Formel (VIII)

OCH-Y¹-Z (VIII)

in denen Z die oben angegebene Bedeutung besitzt und Y¹ eine um eine Methylengruppe verkürzte Alkylenkette Y ist, reduktiv alkyliert [C],
oder mit reaktiven Säurederivaten der allgemeinen Formel (IX)

M-OC-Y¹-Z (IX)

in der Y¹ und Z die oben angegebene Bedeutung haben und M eine in Acylierungsmitteln übliche "leaving group" wie Chlor, Brom, Alkoxy, Aryloxy, Imidazolyl, Thiazolyl, Methansulfonyloxy oder Alkoxycarbonyloxy bedeutet, umsetzt und die erhaltenen Säureamide katalytisch mit Wasserstoff oder mit komplexen Metallhydriden zu Verbindungen der Formel (I) reduziert [D].

Verbindungen der Formel (I) werden auch erhalten,
wenn man
Verbindungen der Formel (X) 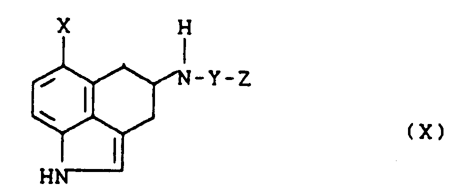
in der X, Y und Z die oben angegebene Bedeutung haben,
mit Alkylierungsmitteln der Formel (XI)

R¹-L (XI)

in der R¹ und L die oben angegebene Bedeutung haben, alkyliert [E],
oder mit Aldehyden der Formel (XII)

R¹³-CHO (XII)

in der R¹³ ein um eine Methylengruppe verkürzter Rest R¹ ist, reduktiv alkyliert [F],
oder mit reaktiven Säurederivaten der allgemeinen Formel (XIII)

M-CO-R¹³ (XIII)

in der R¹³ und M die oben angegebene Bedeutung haben, umsetzt und die erhaltenen Säureamide katalytisch mit Wasserstoff oder mit komplexen Metallhydriden zu Verbindungen der Formel (I) reduziert [G].

Außerdem werden Verbindungen der Formel (I) aus Verbindungen der Formel (VI) erhalten, indem man diese schrittweise zunächst durch Alkylierung oder reduktive Alkylierung zu Zwischenprodukten mit geeigneten funktionellen Gruppen umsetzt und diese Zwischenprodukte dann durch Abwandlung der funktionellen Gruppen durch Oxidation, Reduktion, Hydrolyse oder Umsetzung mit elektrophilen Reagenzien in Verbindungen der Formel (I) überführt [H]

Beispielsweise werden die Verbindungen der Formel (VI) 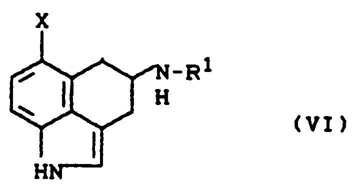
in welcher X und R¹ die oben angegebene Bedeutung haben,
mit Chloracetonitril oder Acrylnitril zu Verbindungen der Formeln (XIV) und (XV) alkyliert, 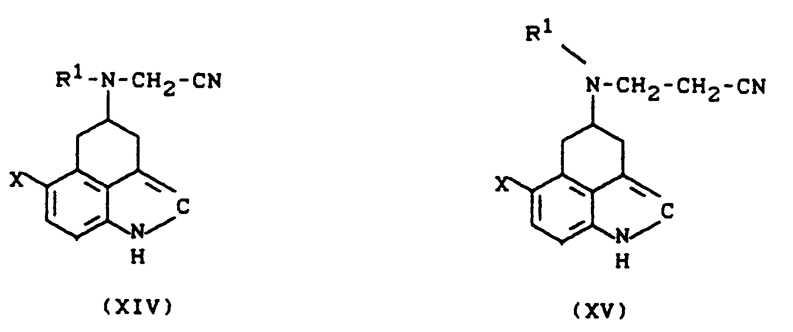
die erhaltenen Nitrile zu den Aminen (XVI) und (XVII) hydriert 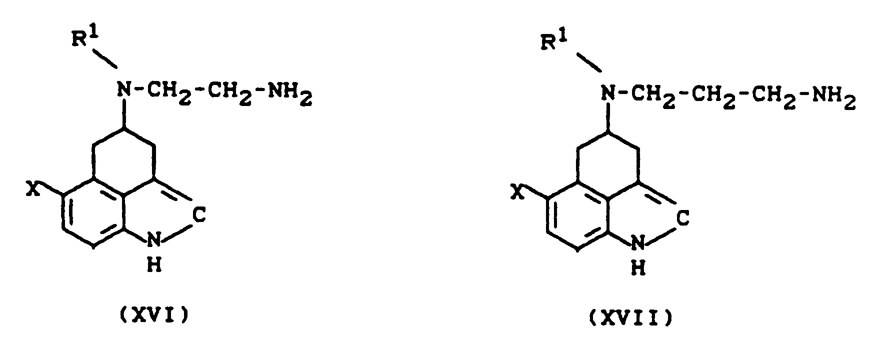
und diese in an sich bekannter Weise durch Alkylierung, reduktive Alkylierung, Acylierung, Umsetzung mit Isocyanaten oder Sulfonylierung in die erfindungsgemäßen Verbindungen der Formel (I) überführt.

Die folgenden Formelschemata sollen die Verfahren zur Herstellung von Verbindungen der Formel (I) beispielhaft erläutern.

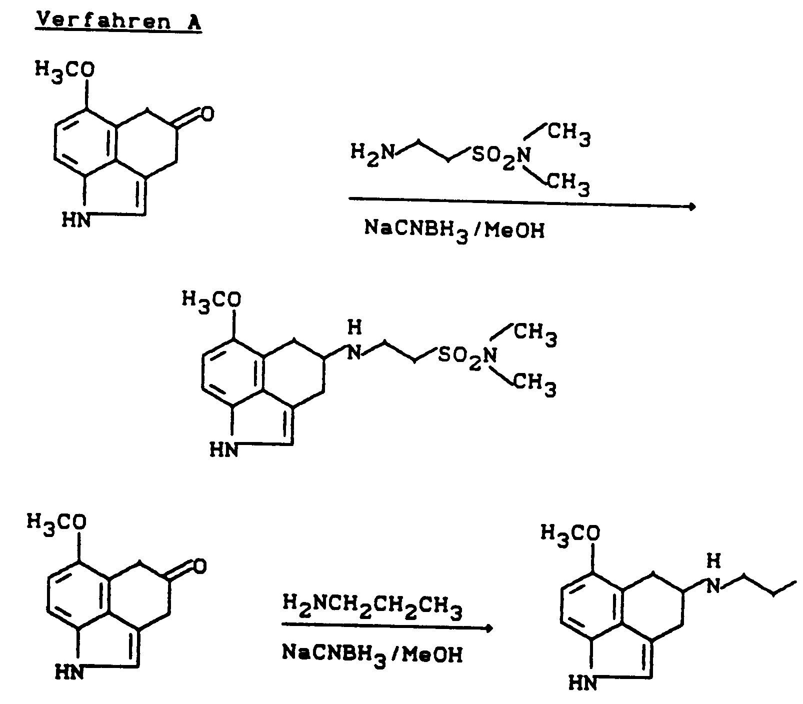

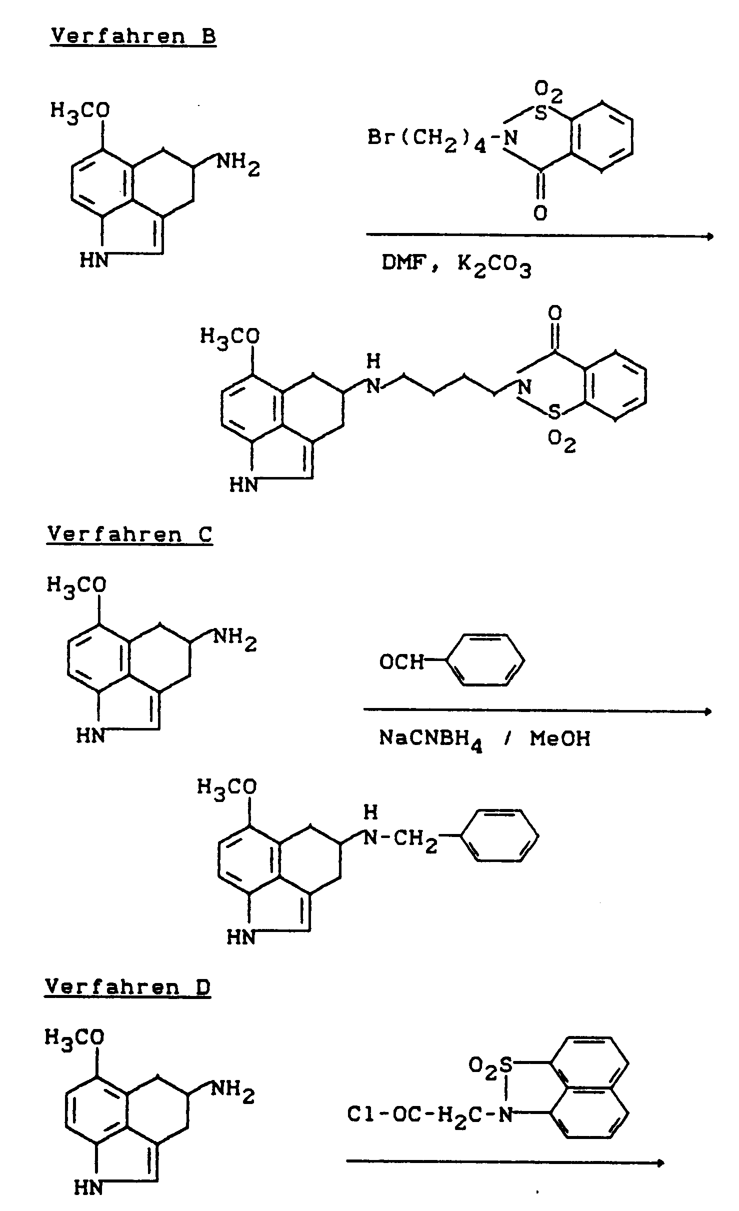

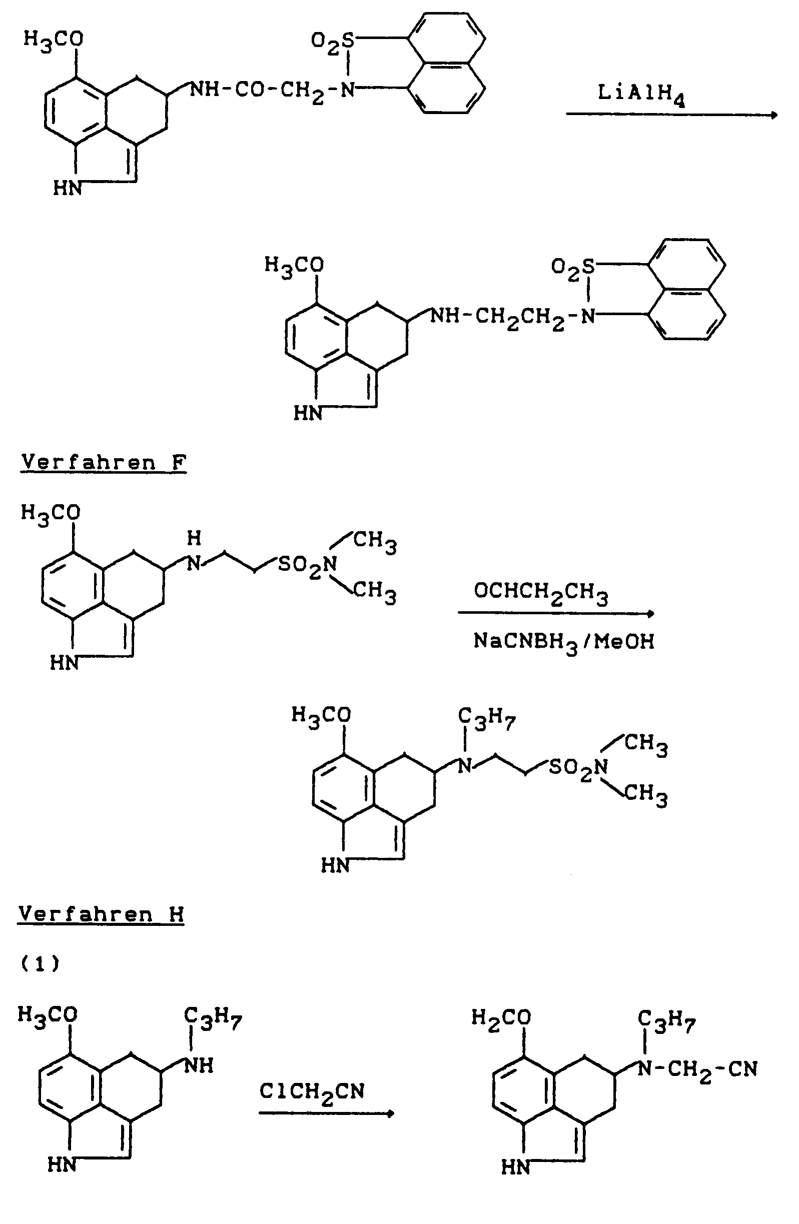

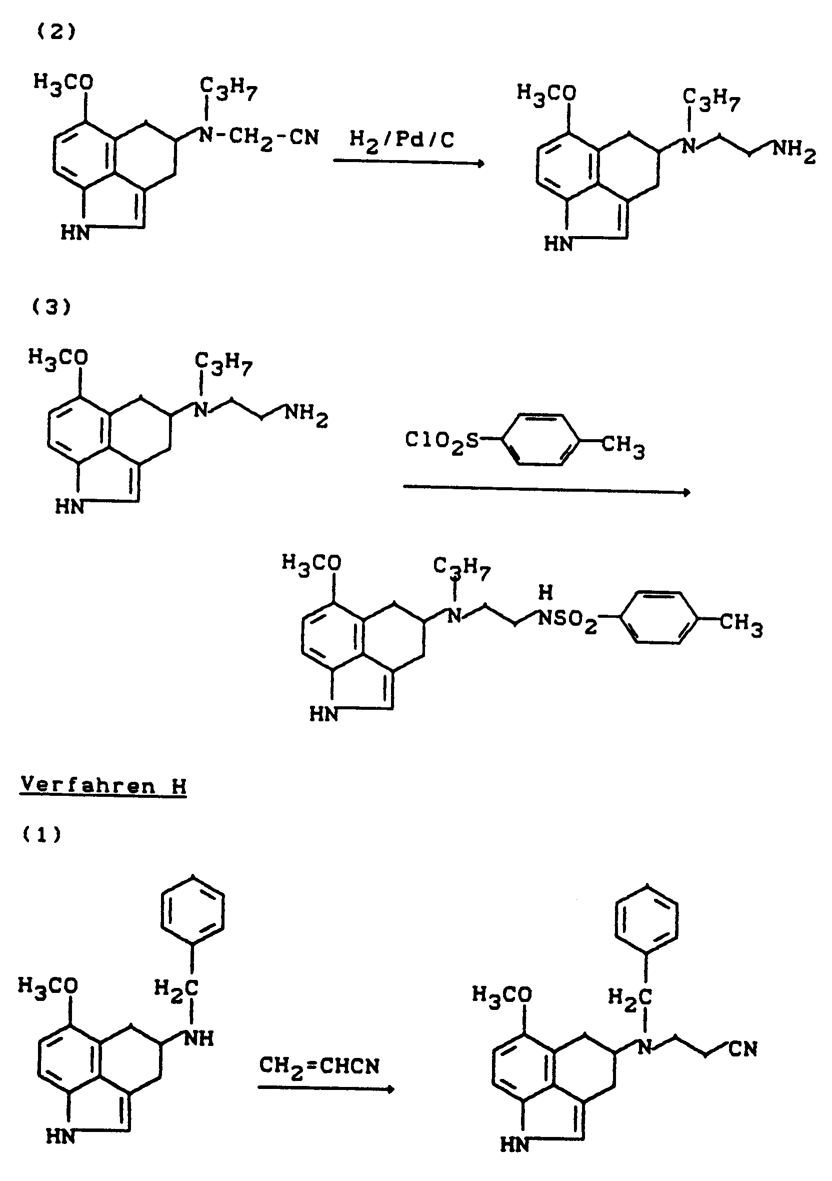

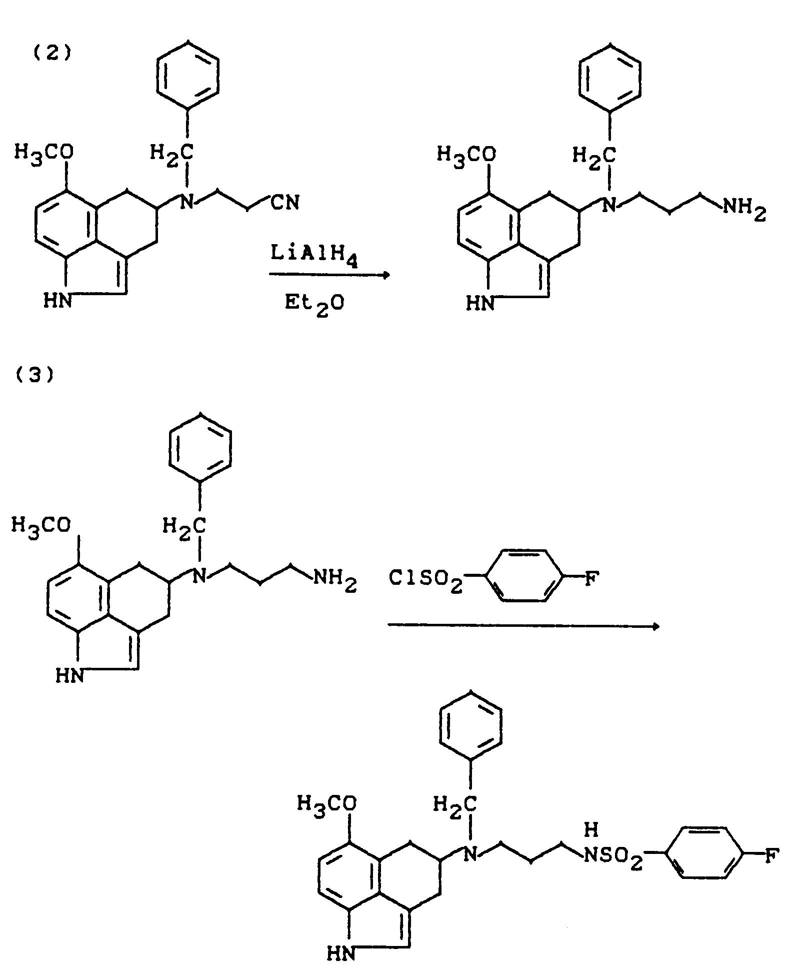

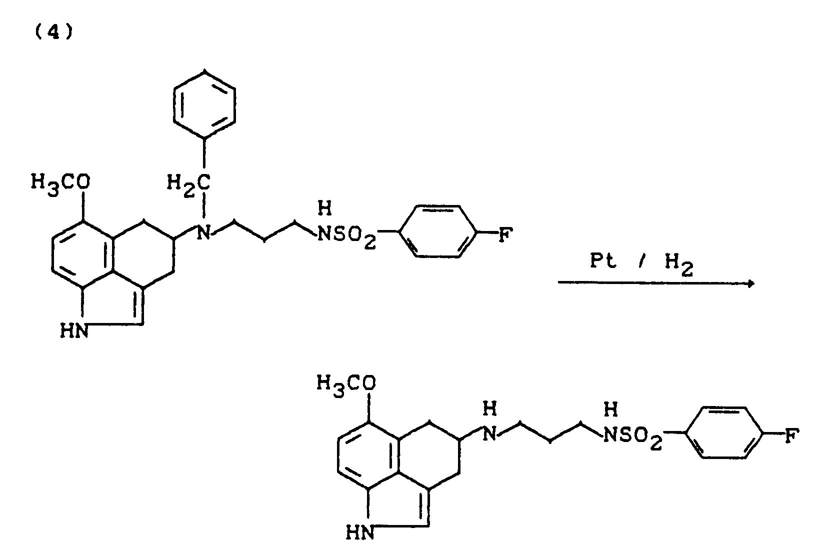

Die als Ausgangsmaterialien verwendeten Ketone der allgemeinen Formel (II) sind bekannt oder können nach bekannten Methoden hergestellt werden [EP 0 162 695 und EP 0 153083].

Die als Ausgangsstoffe verwendeten Amine der allgemeinen Formel (III), (IV) und (V) sind bekannt oder können nach bekannten Methoden hergestellt werden [Houben-Weyl's "Methoden der organischen Chemie" Bd XI/1 und XI/2].

Die Herstellung der Schiff'schen Basen bzw. Enamine durch Umsetzung der Tetralone (II) mit Aminen (III) erfolgt in inerten organischen Lösemitteln, gegebenenfalls in Anwesenheit eines Katalysators und gegebenenfalls in Anwesenheit eines wasserbindenden Mittels.

Das erfindungsgemäße Verfahren kann in zwei Schritten, d.h. mit Isolierung der Enamine durchgeführt werden. Ebenso ist est möglich das erfindungsgemäße Verfahren als Eintopfverfahren durchzuführen.

Als inerte Lösemittel eignen sich hierbei die üblichen organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Butylmethylether, Dioxan, Tetrahydrofuran, Glykoldimethylether oder Diethylenglykoldimethylether, oder Halogenkohlenwasserstoffe wie beispielsweise Methylenchlorid, Chloroform oder Tetrachlorkohlenstoff, oder Kohlenwasserstoffe wie Benzol, Toluol, Xylol, oder Erdölfraktionen, oder Amide wie Dimethylformamid oder Hexamethylphosphorsäuretriamid, oder Essigsäure.
Außerdem ist es möglich, Gemische der genannten Lösemittel zu verwenden.

Als Katalysatoren werden im allgemeinen Säuren verwendet. Hierzu gehören bevorzugt anorganische Säuren wie beispielsweise Salzsäure oder Schwefelsäure, oder organische Sulfon- oder Carbonsäuren wie beispielsweise Methansulfonsäure, Ethansulfonsäure, Benzolsulfonsäure, Toluolsulfonsäure, Essigsäure oder Propionsäure.

Das bei der Reaktion gebildete Wasser kann gegebenenfalls im Gemisch mit dem verwendeten Lösemittel während oder nach der Reaktion z.B. durch Destillation oder durch Zugabe von wasserbindenden Mitteln, wie beispielsweise Phosphorpentoxid oder bevorzugt durch Molekularsieb, entfernt werden.

Die Reaktion wird im allgemeinen in einem Temperaturbereich von 0°C bis + 150°C, bevorzugt von +20°C bis +100°C durchgeführt.

Die Umsetzung kann bei normalem, erhöhtem und bei erniedrigtem Druck durchgeführt werden (z.B. 0,5 - 5 bar). Im allgemeinen arbeitet man bei Normaldruck.

Bei der Durchführung der Reaktion werden im allgemeinen die Ausgangsstoffe in einem molaren Verhältnis von Tetralon (II) zu Amin (III) von 0,5 : 2 bis 1 : 1 eingesetzt. Bevorzugt verwendet man molare Mengen der Reaktanden.

Die Reduktion der Enamine erfolgt entweder durch Wasserstoff in Wasser oder inerten organischen Lösemitteln wie Alkoholen, Ethern oder Halogenkohlenwasserstoffen, oder deren Gemischen, mit Katalysatoren wie Raney-Nickel, Palladium, Palladium auf Tierkohle oder Platin, oder aber mit Hydriden in inerten Lösemitteln, gegebenenfalls in Anwesenheit eines Katalysators.

Bevorzugt wird die Reaktion mit Hydriden, wie komplexen Borhydriden oder Aluminiumhydriden, durchgeführt. Besonders bevorzugt werden hierbei Natriumborhydrid, Lithiumaluminiumhydrid oder Natriumcyanoborhydrid eingesetzt.

Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether oder Diethylenglykoldimethylether, oder Amide wie Hexamethylphosphorsäuretriamid, oder Dimethylformamid oder Essigsäure. Ebenso ist es möglich, Gemische der genannten Lösemittel zu verwenden.

Als Katalysatoren bei der Reduktion mit Natriumcyanoborhydrid werden im allgemeinen Säuren verwendet. Hierzu gehören bevorzugt anorganische Säuren wie beispielsweise Salzsäure oder Schwefelsäure oder organische Carbonsäuren oder Sulfonsäuren, wie beispielsweise Essigsäure, Trifluoressigsäure, Trichloressigsäure, Methansulfonsäure, Ethansulfonsäure, Benzolsulfonsäure oder Toluolsulfonsäure.

Bei der Durchführung des erfindungsgemäßen Verfahrens hat es sich als günstig erwiesen, die Umsetzung der Tetralone (II) mit den Aminen (III) in einem inerten Lösemittel, bevorzugt in Essigester oder in Alkoholen wie beispielsweise Methanol, Ethanol, Propanol oder Isopropanol, oder deren Gemische in Anwesenheit eines Reduktionsmittels, bevorzugt von komplexen Hydriden wie beispielsweise Natriumborhydrid oder Natriumcyanoborhydrid, gegebenenfalls in Anwesenheit eines wasserentziehenden Mittels, bevorzugt von Molsieb, als Eintopfverfahren durchzuführen.

In diesem Fall wird die Reaktion in einem Temperaturbereich von 0°C bis +150°C, bevorzugt von 0°C bis +100°C bei Normaldruck durchgeführt. Es ist ebenso möglich, die Reaktion bei Unterdruck oder bei Überdruck (z.B. im Bombenrohr) durchzuführen.

Wird das erfindungsgemäße Verfahren als Eintopfreaktion durchgeführt, hat es sich als günstig erwiesen, das Amin in einem bis zu 10-fachen, bevorzugt in einem bis zu 5-fachen Überschuß über das Tetralon einzusetzen.

Die Amine der Formel (VI) und (X) sind bekannt (EP 0 162 695 und EP 0 153 083) oder können nach an sich bekannten Verfahren aus den Ketonen der Formel (II) oder Aminen der Formel (VI) (R¹ = H) durch reduktive Aminierung oder durch Alkylierung oder reduktive Alkylierung hergestellt werden.

Als Lösemittel für die Umsetzung der Amine (VI) und (X) mit den Alkylierungsmitteln (VII) und (XI) können hier die üblichen organischen Lösemittel verwendet werden, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether oder Butylmethylether, oder Ketone wie Aceton oder Butanon, oder Amide wie Dimethylformamid oder Hexamethylphosphorsäuretriamid, oder Dimethylsulfoxid, Acetonitril, Essigester, oder Halogenkohlenwasserstoffe wie Methylenchlorid, Chloroform oder Tetrachlorkohlenstoff, oder Pyridin, Picolin oder N-Methylpiperidin. Ebenso können Gemische der genannten Lösemittel verwendet werden.

Als Basen eignen sich die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliummethanolat, oder Natrium- oder Kaliumethanolat, oder organische Amine wie Triethylamin, Picolin oder N-Methylpiperidin, oder Amide wie Natriumamid oder Lithiumdiisopropylamid, oder metallorganische Verbindungen wie Butyllithium oder Phenyllithium.

Die Umsetzung wird im allgemeinen in einem Temperaturbereich von 0°C bis +150°C, bevorzugt von Raumtemperatur bis +80°C durchgeführt.

Die Reaktion wird im allgemeinen bei Normaldruck durchgeführt. Es ist jedoch ebenso möglich, die Reaktion bei erhöhtem oder erniedrigtem Druck durchzuführen.

Als Reaktionsbeschleuniger werden im allgemeinen Alkaliiodide eingesetzt, bevorzugt Natriumiodid oder Kaliumiodid.

Die Base wird hierbei in einer Menge von 1 bis 5, bevorzugt von 1 bis 2 Mol, bezogen auf 1 Mol der Halogenverbindung eingesetzt. Die Halogenverbindung wird bevorzugt in einer bis zu 10-fachen, bevorzugt in einer bis zu 5-fachen Überschußmenge über das alkylsubstituierte 2-Aminotetralin (Ib) eingesetzt.

Die reduktive Alkylierung der Amine (VI) und (X) mit den Aldehyden (VIII) und (XII) erfolgt im allgemeinen in einem Schritt. Ist das Amin (VI) ein primäres Amin kann die Reaktion auch zweistufig durchgeführt werden, wobei zunächst eine Schiff'sche Base bzw. ein Enamin erhalten wird.

Die Herstellung der Schiff'schen Basen bzw. Enamine im ersten Schritt erfolgt in inerten organischen Lösemitteln, gegebenenfalls in Anwesenheit eines Katalysators und gege benenfalls in Anwesenheit eines wasserbindenden Mittels. Das erfindungsgemäße Verfahren kann in 2 Schritten, d.h. mit Isolierung der Zwischenprodukte durchgeführt werden. Ebenso ist es möglich die Reduktion als Eintopfverfahren durchzuführen.

Als inerte Lösemittel eignen sich hierbei die üblichen organischen Lösemittel, die sich unter den Reaktionsbedingungen nicht verändern, Hierzu gehören bevorzugt Alkohole wie Methanol, Ethanol, Propanol oder Isopropanol, oder Ether wie Diethylether, Butylmethylether, Dioxan, Tetrahydrofuran, Glykoldimethylether oder Diethylenglykoldiethylether, oder Halogenkohlenwasserstoffe wie beispielsweise Methylenchlorid, Chloroform oder Tetrachlorkohlenstoff, oder Kohlenwasserstoffe wie Benzol, Toluol, Xylol, oder Erdölfraktionen, oder Amide wie Dimethylformamid oder Hexamethylphosphorsäuretriamid oder Essigsäure. Außerdem ist es möglich, Gemische der genannten Lösemittel zu verwenden.

Als Katalysatoren werden im allgemeinen Protonensäuren verwendet. Hierzu gehören bevorzugt anorganische Säuren wie beispielsweise Salzsäure oder Schwefelsäure, oder organische Carbonsäuren mit 1-6 C-Atomen, gegebenenfalls substituiert durch Fluor, Chlor und/oder Brom, wie beispielsweise Essigsäure, Trifluoressigsäure, Trichloressigsäure oder Propionsäure, oder Sulfonsäuren mit C₁-C₄-Alkylresten oder mit Arylresten wie beispielsweise Methansulfonsäure, Ethansulfonsäure, Benzolsulfonsäure oder Toluolsulfonsäure.

Das bei der Reaktion gebildete Wasser kann gegebenenfalls im Gemisch mit dem verwendeten Lösemittel während oder nach der Reaktion z.B. durch Destillation oder durch Zugabe von wasserbindenden Mitteln, wie beispielsweise Phosphorpentoxid oder bevorzugt durch Molekularsieb entfernt werden.

Die Reaktion wird im allgemeinen in einem Temperaturbereich von 0°C bis 150°C, bevorzugt von +20°C bis +100°C durchgeführt.

Die Umsetzung kann bei normalem, erhöhtem und bei erniedrigtem Druck durchgeführt werden (z.B. 0,5 - 5 bar). Im allgemeinen arbeitet man bei Normaldruck.

Bei der Durchführung wird die Verbindung (V) in einer Menge von 0,1 - 10, bevorzugt von 0,5 - 5 mol, bezogen auf 1 mol monosubstituiertes basisches 2-Aminotetralin (Ic) eingesetzt.

Die Reduktion der Schiff'schen Basen bzw. Enamine im zweiten Schritt erfolgt entweder durch Wasserstoff in Wasser oder in inerten organischen Lösemitteln wie Alkoholen, Ethern oder Halogenkohlenwasserstoffen, oder deren Gemischen, mit Katalysatoren wie Raney-Nickel, Palladium, Palladium auf Tierkohle oder Platin, oder mit Hydriden in inerten Lösemitteln, gegebenenfalls in Anwesenheit eines Katalysators.

Bevorzugt wird die Reaktion mit Hydriden, wie komplexen Borhydriden oder Aluminiumhydriden durchgeführt. Besonders bevorzugt werden hierbei Natriumborhydrid, Lithiumaluminiumhydrid oder Natriumcyanoborhydrid eingesetzt.

Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether oder Amide wie Hexamethylphosphorsäuretriamid oder Dimethylformamid, oder Essigsäure. Ebenso ist es möglich, Gemische der genannten Lösemittel zu verwenden.

Als Katalysatoren bei der Reduktion mit Natriumcyanoborhydrid werden im allgemeinen Protonensäuren verwendet. Hierzu gehören bevorzugt anorganische Säuren wie beispielsweise Salzsäure oder Schwefelsäure, oder organische Carbonsäure mit 1-6 C-Atomen, gegebenenfalls substituiert durch Fluor, Chlor und/oder Brom, wie beispielsweise Essigsäure, Trifluoressigsäure, Trichloressigsäure oder Propionsäure, oder Sulfonsäuren mit C₁-C₄-Alkylresten oder mit Arylresten wie beispielsweise Methansulfonsäure, Ethansulfonsäure, Benzolsulfonsäure oder Toluolsulfonsäure.

Bei der Durchführung des erfindungsgemäßen Verfahrens hat es sich als günstig erwiesen, die Umsetzung der Aldehyde (VIII) und (XII) mit den Aminen (VI) und (X) in einem inerten Lösemittel, bevorzugt in Essigsäure oder Alkoholen wie beispielsweise Methanol, Ethanol, Propanol oder Isopropanol oder deren Gemischen, in Anwesenheit von anorganischen oder organischen Säuren wie beispielsweise Chlorwasserstoffsäure oder Essigsäure, und in Anwesenheit eines Reduktionsmittels, bevorzugt von komplexen Hydriden wie beispielsweise Natriumborhydrid oder Natriumcyanoborhydrid, gegebenenfalls in Anwesenheit eines wasserentziehenden Mittel, bevorzugt Molekularsieb, als Eintopfverfahren durchzuführen.

In diesem Fall wird die Reaktion in einem Temperaturbereich von 0°C bis +150°C, bevorzugt von 0°C bis +100°C bei Normaldruck durchgeführt. Es ist ebenso möglich die Reaktion bei Unterdruck oder bei Überdruck (z.B. im Bombenrohr) durchzuführen.

Das Überführen von funktionellen Gruppen in andere funktionelle Gruppen in den oben aufgeführten Herstellungsverfahren erfolgt je nach Art der funktionellen Gruppen durch Oxidation, Reduktion, Hydrolyse oder durch Umsetzung mit elektrophilen Reagenzien und soll im folgenden erläutert werden.
1. Die Reduktion der Nitrilgruppe zur Aminogruppe erfolgt im allgemeinen mit Metallhydriden, bevorzugt mit Lithiumaluminiumhydrid, Aluminiumhydrid (hergestellt z.B. durch Umsetzung von Lithiumaluminumhydrid mit 100%iger Schwefelsäure oder mit Aluminiumchlorid) oder deren Gemischen in inerten Lösemitteln wie Ethern oder Chlorkohlenwasserstoffen, bevorzugt in Ethern wie beispielsweise Tetrahydrofuran, Diethylether oder Dioxan in einem Temperaturbereich von -20°C bis +100°C, bevorzugt von 0°C bis +50°C bei Normaldruck.
   Die Reduktion ist außerdem durch Hydrieren der Nitrile in inerten Lösemitteln wie Alkoholen z.B. Methanol, Ethanol, Propanol oder Isopropanol in Gegenwart eines Edelmetallkatalysators wie Platin, Palladium, Palladium auf Tierkohle oder Raney-Nickel, in einem Temperaturbereich von 0°C bis +150°C, bevorzugt von Raumtemperatur bis +100°C bei Normaldruck oder bei Überdruck möglich.
   Die Reaktion kann durch folgendes Schema verdeutlicht werden: 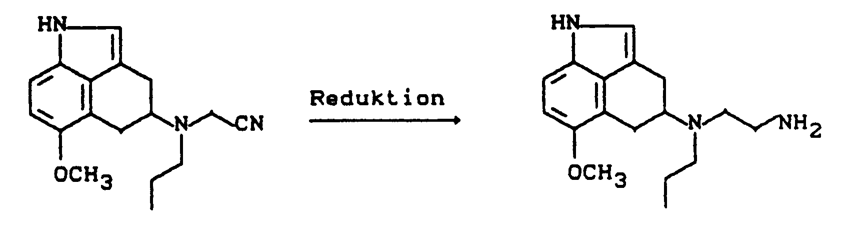
2. Die Reduktion von Alkoxycarbonylgruppen in Alkoholgruppen erfolgt im allgemeinen mit Hydriden, bevorzugt mit Lithiumaluminiumhydrid in inerten Lösemitteln wie Ethern, Kohlenwasserstoffen oder Halogenkohlenwasserstoffen oder deren Gemischen, bevorzugt in Ethern wie beispielsweise Diethylether, Tetrahydrofuran oder Dioxan in einem Temperaturbereich von 0°C bis +150°C, bevorzugt von +20°C bis +100°C bei Normaldruck.
   Die Reaktion kann durch folgendes Schema verdeutlicht werden: 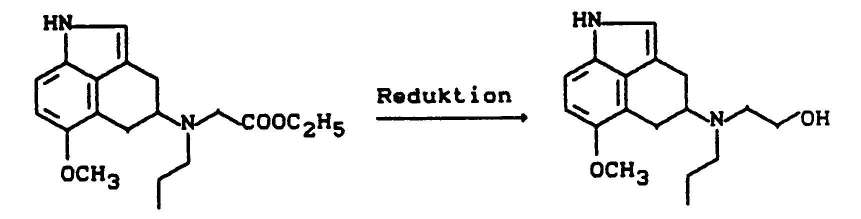
3. Die Hydrolyse der Nitrilgruppe zur Carbonamidgruppe erfolgt im allgemeinen mit Hilfe von starken Mineralsäuren, bevorzugt mit Chlorwasserstoff in inerten Lösemitteln wie Wasser und/oder Alkoholen wie beispielsweise Methanol, Ethanol, Propanol oder Isopropanol in einem Temperaturbereich von 0°C bis +150°C, bevorzugt von +20°C bis +100°C bei Normaldruck.
   Die Reaktion kann durch folgendes Schema erläutert werden: 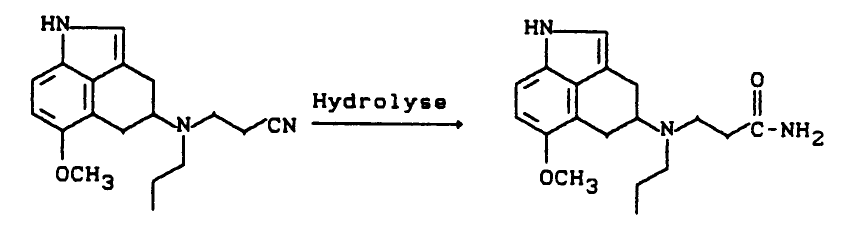
4. Durch Umsetzung von NH- oder OH-aciden Verbindungen (Z in Formel (I) ist gleich OH oder NR²R³, wobei R² = H und R³ = H, Alkyl, Aryl oder Aralkyl ist), mit elekrophilen Reagenzien erhält man eine Vielzahl weiterer erfindungsgemäßer Verbindungen:
   a) Die Überführung von Aminen in Carbonamide erfolgt im allgemeinen durch Umsetzung mit Carbonsäureestern in inerten Lösemitteln wie Ethern oder deren Gemischen oder Kohlenwasserstoffen, bevorzugt in Ethern wie beispielsweise Diethylether, Tetrahydrofuran oder Dioxan, gegebenenfalls in Anwesenheit von Basen wie Alkalimetalle, Alkalihydride, Alkalialkoholate oder lithiumorganischen Verbindungen, bevorzugt in Anwesenheit von Alkalimetallen wie beispielsweise Natrium oder Alkalihydriden wie Natriumhydrid oder Kaliumhydrid in einem Temperaturbereich von +20°C bis +150°C, bevorzugt bei der Siedetemperatur des verwendeten Lösemittels bei Normaldruck.
      Darüberhinaus ist es möglich, die Amide mit Carbonsäurehalogeniden oder -anhydriden, bevorzugt mit Carbonsäurechloriden in inerten Lösemitteln wie Ethern, Kohlenwasserstoffen oder Halogenkohlenwasserstoffen oder deren Gemischen, bevorzugt in Ethern wie beispielsweise Diethylether, Tetrahydrofuran, oder Halogenkohlenwasserstoffen wie Methylenchlorid oder Chloroform, gegebenenfalls in Anwesenheit von Basen wie Alkalicarbonaten beispielsweise Natriumcarbonat oder Kaliumcarbonat, oder organischen Aminen wie beispielsweise Triethylamin oder Pyridin, in einem Temperaturbereich von -20°C bis +100°C, bevorzugt von 0°C bis +60°C bei Normaldruck herzustellen.
      Die Reaktion kann durch folgendes Schema verdeutlicht werden: 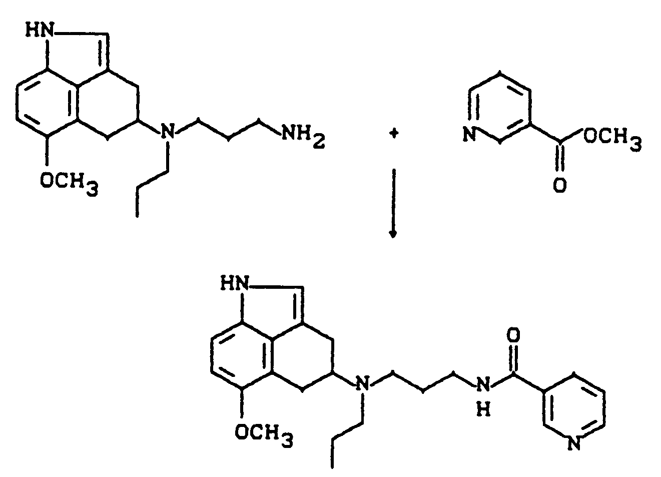
   b) Die Überführung von Aminen in Carbamate erfolgt im allgemeinen mit Kohlensäureestern, bevorzugt mit Kohlensäureestern, die einen Phenylesterrest tragen oder mit Chlorkohlensäureestern, in inerten Lösemitteln wie Ethern, Kohlenwasserstoffen oder Halogenkohlenwasserstoffen oder deren Gemischen, bevorzugt in Ethern wie beispielsweise Diethylether, Tetrahydrofuran oder Dioxan, in einem Temperaturbereich von +20°C bis +150°C, bevorzugt von +20°C bis +100°C bei Normaldruck. Die Reaktion kann auch in einem Zweiphasensystem durchgeführt werden, wobei die wäßrige Phase eine Hilfsbase wie Natrium-oder Kaliumcarbonat oder -hydrogencarbonat enthält.
      Die Reaktion kann durch folgendes Schema erläutert werden: 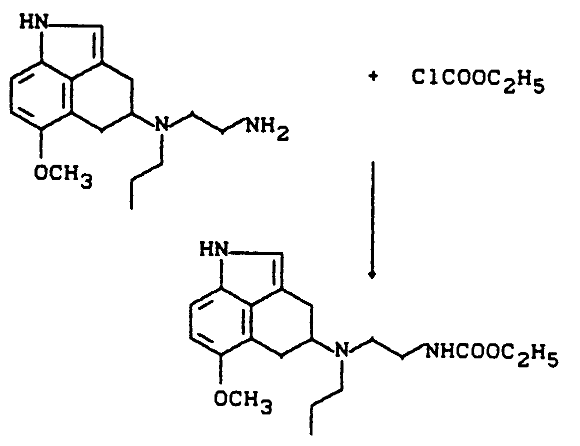
   c) Die Überführung von Aminen in Harnstoffe erfolgt im allgemeinen durch Umsetzung mit Isocyanaten in inerten Lösemitteln wie Ethern, Kohlenwasserstoffen oder Halogenkohlenwasserstoffen oder deren Gemischen, bevorzugt in Ethern wie beispielsweise Diethylether oder Tetrahydrofuran, oder in Halogenkohlenwasserstoffen wie beispielsweise Methylenchlorid oder Chloroform, in einem Temperaturbereich von -20°C bis +150°C, bevorzugt von 0°C bis +100°C bei Normaldruck.
      Die Reaktion kann durch folgendes Schema erläutert werden: 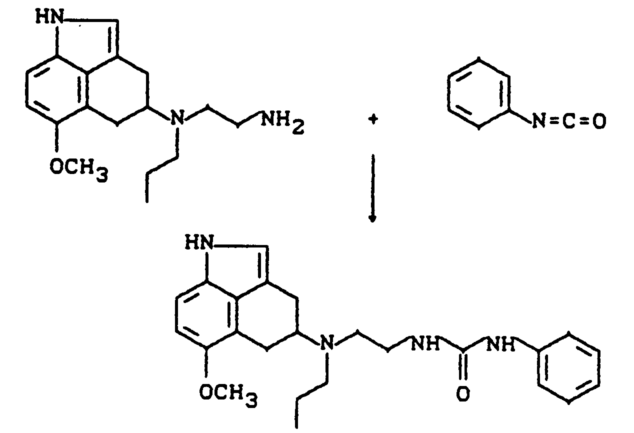
   d) Die Überführung von Aminen in Sulfonamide bzw. Aminosulfamoylderivate erfolgt im allgemeinen mit Sulfonsäurehalogeniden bzw. mit Amidosulfonsäurehalogeniden, bevorzugt mit den entsprechenden Chloriden in inerten Lösemitteln wie Ethern, Kohlenwasserstoffen oder Halogenkohlenwasserstoffen oder deren Gemische, bevorzugt in Halogenkohlenwasserstoffen wie beispielsweise Methylenchlorid oder Chloroform, gegebenenfalls in Anwesenheit von Basen wie Alkalihydroxide, Alkalicarbonate, Alkalialkoholate oder organische Amine, bevorzugt mit Alkalihydroxiden wie Natriumhydroxid oder Kaliumhydroxid, Alkalicarbonaten wie beispielsweise Natriumcarbonat oder Kaliumcarbonat, oder organische Aminen wie Triethylamin oder Pyridin, in einem Temperaturbereich von -20°C bis +100°C, bevorzugt von 0°C bis +50°C bei Normaldruck.
      Die Reaktion kann durch folgendes Schema verdeutlicht werden: 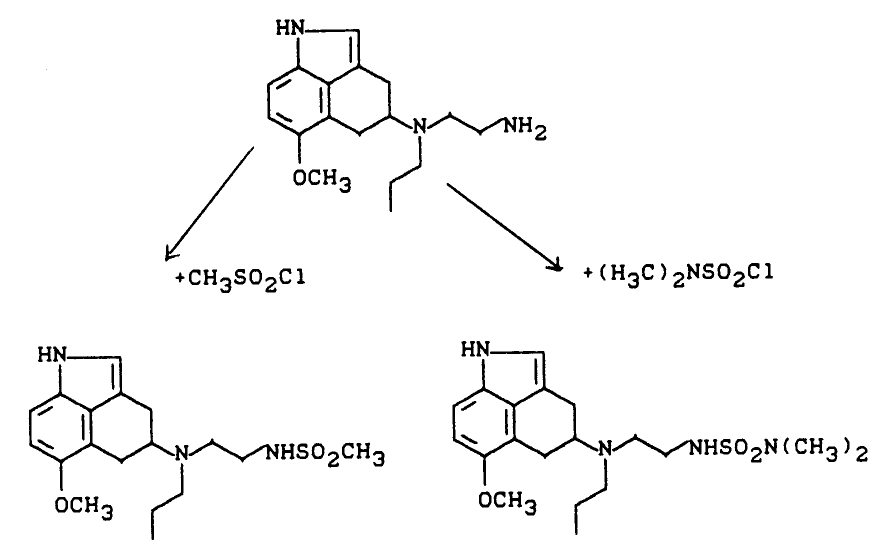
   e) Die Überführung der Hydroxygruppe in Kohlensäureester erfolgt im allgemeinen durch Umsetzen mit Halogenameisensäureestern, bevorzugt mit Chlorameisensäureestern in inerten Lösemitteln wie Ethern, Kohlenwasserstoffen oder Halogenkohlenwasserstoffen, bevorzugt in Halogenkohlenwasserstoffen wie Methylenchlorid oder Chloroform, oder in Ethern wie Diethylether oder Tetrahydrofuran, gegebenenfalls in Anwesenheit von Basen wie Alkylihydroxide, Alkalicarbonate oder organischen Aminen, bevorzugt in Anwesenheit von organischen Aminen wie Triethylamin, Pyridin, Picolin oder Dimethylaminopyridin in einem Temperaturbereich von -20°C bis +100°C, bevorzugt von 0°C bis Raumtemperatur bei Normaldruck.
      Die Reaktion läßt sich durch folgendes Schema verdeutlichen: 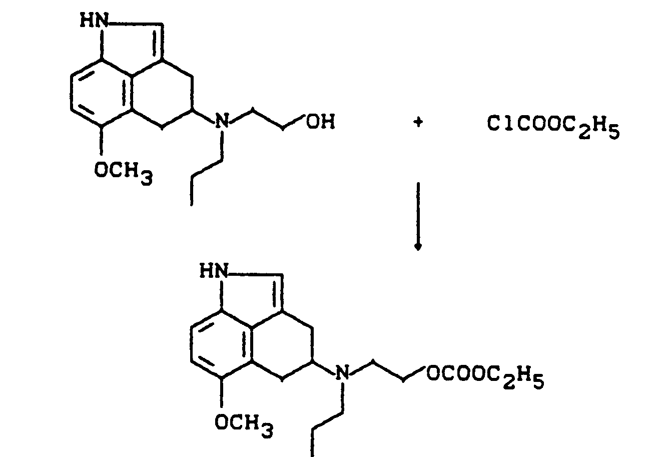
   f) Cyclische Sulfonamide werden im allgemeinen durch Reaktion intramolekularer Elektrophile in inerten dipolar aprotischen Lösemitteln bevorzugt in Dimethylformamid, Hexamethylphosphorsäuretriamid oder Dimethylsulfoxid, gegebenenfalls in Anwesenheit von Basen wie Alkalimetalle, Alkalihydride, Alkaliamide, Alkalialkoholate oder lithiumorganischen Verbindungen, bevorzugt in Anwesenheit von Alkalihydriden wie Natriumhydrid oder Kaliumhydrid, oder Alkaliamiden wie Natriumamid oder Lithiumdiisopropylamid, gegebenenfalls in Gegenwart katalytischer Mengen eines Alkaliiodides beispielsweise Natriumiodid oder Kaliumiodid in einem Temperaturbereich von -20°C bis +100°C, bevorzugt von 0°C bis +50°C bei Normaldruck hergestellt.
      Die Reaktion läßt sich durch folgendes Schema verdeutlichen: 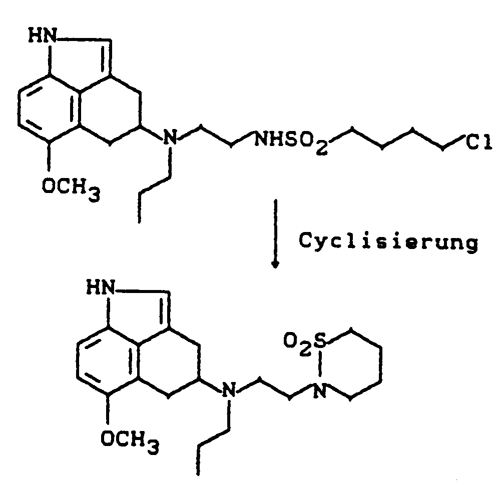
5. Die Oxidation der Thioethergruppe zu Sulfoxiden bzw. Sulfonen erfolgt im allgemeinen mit Oxidationsmitteln wie Peroxoverbindungen oder Wasserstoffperoxid selbst, bevorzugt mit Wasserstoffperoxid, in inerten Lösemitteln wie Carbonsäuren und Carbonsäureanhydriden, bevorzugt in Essigsäure, in einem Temperaturbereich von -20°C bis +100°C, bevorzugt von 0°C bis +50°C.
   Die Reaktion läßt sich durch folgendes Schema verdeutlichen:

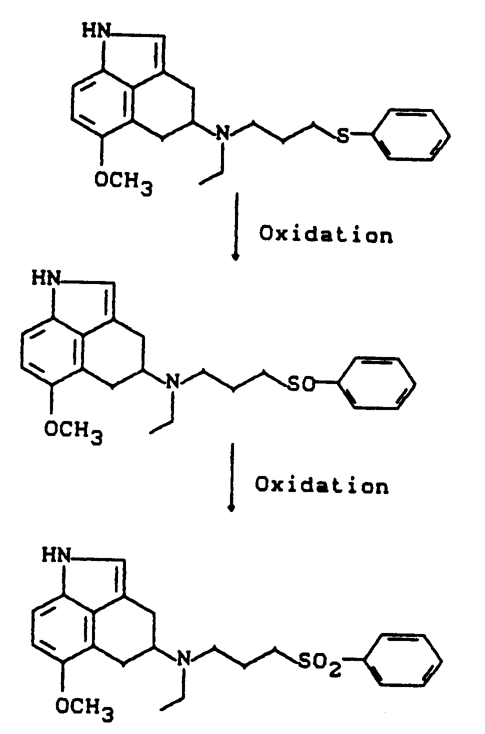

Die als Ausgangsstoffe eingesetzten Amine der allgemeinen Formel (III), (IV) und (V) sind bekannt oder können nach bekannten Methoden hergestellt werden [Houben-Weyl's "Methoden der organischen Chemie" Bd XI/1 und XI/2].

Als Amine können beispielsweise erfindungsgemäß verwendet werden:
Ammoniak, Methylamin, Ethylamin, Propylamin, Isopropylamin, Butylamin, 4-Dimethylaminobutylamin, 4-Diethylaminobutylamin, 3-Dimethylaminopropylamin, 3-Diethylaminopropylamin, 2-Dimethylaminoethylamin, 2-Diethylaminoethylamin, 2-Amino-1-ethoxycarbonylamido-ethan, 3-Amino-1-ethoxycarbonylamido-propan, 4-Amino-1-ethoxycarbonylamido-butan, 3-Aminochinuclidin, 2-[(Phenylaminocarbonyl)amino]ethylamin, 2-[(Phenylaminocarbonyl)amino]propylamin, 4-Aminomethyl-piperidin, 4-(Ethoxycarbonyl)amino-ethyl-piperidin, N-Methylpiperazin, 4-Amino-1-carboxyethyl-piperidin, N,N-Dimethylpropyliden-diamin, N,N-Diethylpropyliden-diamin, N,N-Diethylethyliden-diamin, N,N-Dimethylethylen-diamin, N-(2-Aminoethyl)ethylcarbamat, N-(2-Aminoethyl)propylcarbamat.

Die Halogenverbindungen der allgemeinen Formeln (VII) und (XI) sind bekannt oder können nach bekannten Methoden hergestellt werden [Beilstein's Handbuch der organischen Chemie 2, 197, 201, 250, 278; 3, 9, 10; 21, 461, 462, 463].

Als Halogenverbindungen können beispielsweise erfindungsgemäß verwendet werden:
Chloracetonitril, 2-Chlorpropionitril, 3-Chlorbutyronitril, 3-Brompropylphthalimid, 3-Chlorpropylphthalimid, 2-Bromethylphthalimid, 2-Bromethylphthalimid, 4-Brombutylphthalimid, 4-Chlorbutylphthalimid, Chloressigsäurediethylamid, Chloressigsäuredimethylamid, Chloressigsäuremethylester, Chloressigsäureethylester, Bromessigsäureethylester, Bromessigsäuremethylester, 2-δ-Brombutyl-1,2-benzoisothiazol-3(2H)-on-1,1-dioxid, 2-γ-Brompropyl-1,2-benzoisothiazol-3(2H)-on-1,1-dioxid.

Die als Ausgangsstoffe eingesetzten Carbonylverbindungen der allgemeinen Formeln (VIII) und (XII) sind bekannt oder können nach bekannten Methoden hergestellt werden [Beilstein's Handbuch der organischen Chemie 1, 594, 629, 662].

Als Aldehyde können beispielsweise erfindungsgemäß verwendet werden:
Acetaldehyd, Propionaldehyd, Butyraldehyd, Benzaldehyd.

Die erfindungsgemäßen Verbindungen können als Wirkstoffe in Arzneimitteln verwendet werden.
Die erfindungsgemäßen Stoffe haben eine besonders hohe Affinität zu cerebralen 5-Hydroxy-tryptamin-Rezeptoren vom 5-HT₁-Typ.
Hiermit verbunden sind agonistisch, partiell agonistische oder antagonistische Wirkungen am Serotonin-Rezeptor. Im Vergleich zu den strukturverwandten bekannten Verbindungen weisen sie überraschenderweise eine größere therapeutische Breite auf.

Die in der vorliegenden Erfindung beschriebenen hochaffinen Liganden für den Serotonin-1-Rezeptor stellen somit Wirkstoffe zur Bekämpfung von Krankheiten dar, die durch Störungen des serotoninergen Systems, insbesondere bei Involvierung von Rezeptoren, die hohe Affinität zu 5-Hydroxytryptamin besitzen (5-HT₁-Typ), gekennzeichnet sind. Sie eignen sich daher zur Behandlung von Erkrankungen des Zentralnervensystems wie Angst-, Spannungs- und Depressionszuständen, zentralnervös bedingten Sexualdysfunktionen, Schlafstörungen, sowie zur Behandlung kognitiver Defizite zur Verbesserung von Lern- und Gedächtnisleistungen und zur Behandlung der Alzheimer'schen Krankheit. Weiterhin eignen sich diese Wirkstoffe auch zur Modulierung des cardiovaskulären Systems. Sie greifen auch in die Regulation der cerebralen Durchblutung ein und stellen somit wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall oder cerebraler Ischämie. Ebenso können die erfindungsgemäßen Verbindungen zur Bekämpfung von Schmerzzuständen eingesetzt werden. Auch eignen sie sich zur Bekämpfung von Erkrankungen des Intestinaltraktes, die durch Störungen des serotoninergen Systems wie auch durch Störungen des Kohlenhydrathaushaltes gekennzeichnet sind.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:
Wasser, nicht-toxische organische Lösungsmittel, wie Paraffine (z.B. Erdölfraktionen), pflanzliche Öle (z.B. Erdnuß/Sesamöl), Alkohole (z.B: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester), Polyoxyethylen-Fettalkohol-Ether (z.B. Ligninsulfitablaugen, Methylcellulose, Stärke und Polyvinylpyrrolidon) und Gleitmittel (z.B. Magnesiumstearat, Talkum, Stearinsäure und Natriumsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Herstellungsbeispiele

### Beispiel 1

### 6-Methoxy-4-[4-(N-1,2,-benzisothiazol-3(2H)-on-1,1-dioxidyl)]-butylamino-1,3,4,5-tetrahydrobenz[c,d]indol Hydrochlorid

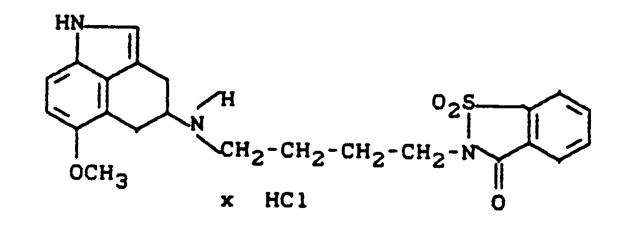

2,5 g 6-Methoxy-4-amino-1,3,4,5-tetrahydrobenz[c,d]-indol und 1,3 g Triethylamin werden in 50 ml Dimethylformamid vorgelegt. Man tropft eine Lösung aus 2-(4-Brombutyl)-1,2-benzisothiazol-3(2H)-on-1,1-dioxid in 20 ml Dimethylformamid zu und rührt 4 Stunden bei 50°C nach. Man gießt auf Wasser und extrahiert mit Methylenchlorid. Das Rohprodukt wird über Kieselgel 60 (40-63 µm) mit Essigester : Ethanol 9:1 zweimal chromatographiert. Nach der zweiten Chromatographie werden die sauberen Fraktionen vereinigt, mit etherischer HCl versetzt und bei Raumtemperatur das Lösemittel abgezogen. Man erhält die Titelverbindung als pulverförmiges Hydrochlorid.
- DC:: (Kieselgel 60) Diisopropylether: Ethanol 3:2
- R_{f} :: 0.458
- Ausbeute:: 2,5 g (40,5% der Theorie)

### Beispiel 2

### 4-[4-(N-1,2-benzisothiazol-3(2H)-on-1,1-dioxid-yl)]-butylamino-1,3,4,5-tetrahydrobenz[c,d]-indol

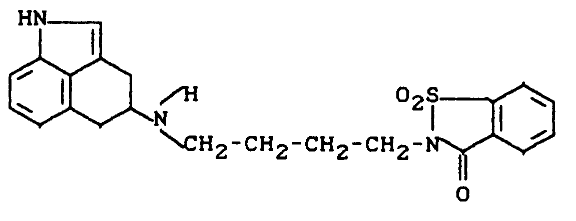

1,72 g 4-Amino-1,3,4,5-tetrahydrobenz[c,d]-indol und 1,01 g Triethylamin werden in 40 ml Dimethylformamid vorgelegt. Man tropft eine Lösung aus 3,18 g 2-(4-Brombutyl)-1,2-benzisothiazol-3(2H)-on-1,1-dioxid in 15 ml Dimethylformamid zu und rührt 4 Stunden bei 50°C nach. Man gießt auf Wasser und extrahiert mit Methylenchlorid. Das Rohprodukt wird über Kieselgel 60 (40-63 µm) mit Essigester:Ethanol 9:1 chromatographiert. Die sauberen Fraktionen werden vereinigt, mit etherischer Salzsäure versetzt, das Lösemittel wird abgezogen und die Substanz als pulverförmiges Hydrochlorid isoliert.
- DC:: (Kieselgel 60) Essigester : Ethanol 9:1
- R_{f} :: 0,142
- Ausbeute:: 2,35 g (52,7% der Theorie)

### Beispiel 3

### 6-Methoxy-4-[4-(N-2,3-dihydro-1,2-benzisothiazol-1,1,-dioxydyl)]-butylamino-1,3,4,5-tetrahydrobenz[c,d]indolhydrochlorid

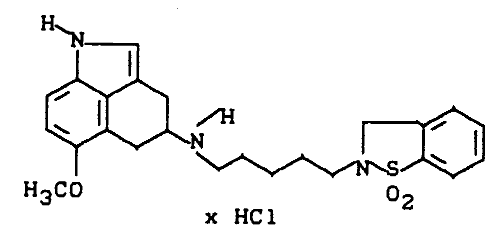

Diese Verbindung wurde analog dem im Beispiel 1 beschriebenen Verfahren hergestellt, wobei 6-Methoxy-4-amino-1,3,4,5-tetrahydrobenz[c,d]-indol und 2-(4-Brombutyl)-2,3-dihydro-1,2-benzisothiazol-1,1-dioxid als Ausgangsprodukte verwendet wurden.
- Fp.:: 234-237°C
- DC :: (Kieselgel 60) Toluol/Methanol 7:3
- R_{f} :: 0,27
- Ausbeute:: 29 % der Theorie.

### Beispiel 4

### 6-Methoxy-4-[4-(N-4-fluorbenzolsulfonyl-N-methyl)-amidobutyl]-amino-1,3,4,5-tetrahydrobenz[c,d]indolhydrochlorid

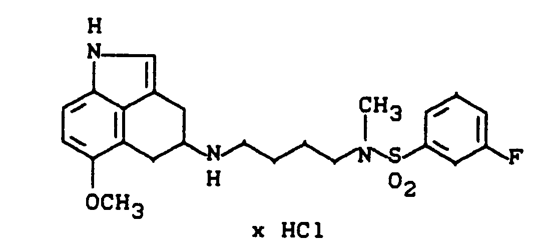

Diese Verbindung wurde analog dem im Beispiel 1 beschriebenen Verfahren hergestellt, wobei 6-Methoxy-4-amino-1,3,4,5-tetrahydrobenz[c,d]-indol und N-Methyl-N-(4-brombutyl)-4-fluorbenzsulfonamid als Ausgangsprodukte verwendet wurden.
- Fp.:: 168-170°C
- DC :: (Kieselgel 60) Toluol/Methanol 7:3
- R_{f} :: 0,32
- Ausbeute:: 36 % der Theorie.

### Beispiel 5

### 6-Methoxy-4-[N-propyl-N-(ethyloxycarbonylaminoethyl)]-amino-1,3,4,5-tetrahydrobenz[c,d]-indol

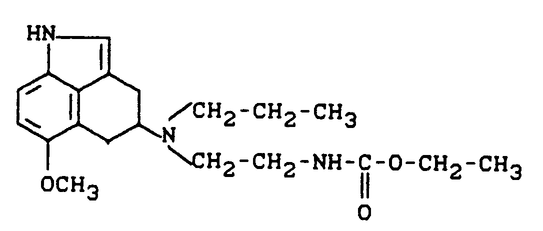

660 mg 6-Methoxy-4-[N-propyl-N-(aminoethyl)]-amino-1,3,4,5-tetrahydrobenz[c,d]-indol werden in 25 ml Methylenchlorid vorgelegt. Man gibt 5 ml 50%ige wässrige Kaliumcarbonatlösung zu. Unter kräftigem Rühren tropft man bei 0°C eine Lösung aus 236 µl Chlorameisensäureethylester und 5 ml Methylenchlorid zu. Man rührt 1 Stunde bei 0°C nach. Man verdünnt mit Wasser und trennt die Phasen. Es wird noch einmal mit Methylenchlorid extrahiert. Die organischen Phasen werden vereinigt, über Natriumsulfat getrocknet und filtriert. Die Lösung wird mit 1,5 g A-Kohle versetzt und 2 Stunden bei Raumtemperatur gerührt. Man filtriert und dampft ein. Man erhält die Substanz als zähes Harz.
- DC :: (Kieselgel 60) Diisopropylether : Ethanol 3:2
- R_{f} :: 0,606
- Ausbeute:: 540 mg (64% der Theorie)

### Beispiel 6

### 6-Methoxy-4-[N-propyl-N-(methylsulfonylamidoethyl)]-amino-1,3,4,5-tetrahydrobenz[c,d]-indol

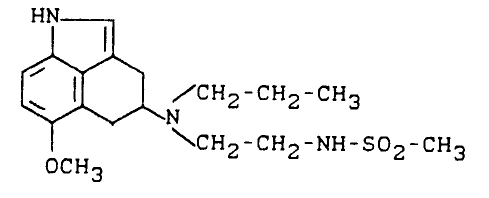

1,14 g 6-Methoxy-4-[N-propyl-N-(aminoethyl)]-amino-1,3,4,5-tetrahydrobenz[c,d]-indol und 553 mg Kaliumcarbonat werden in 40 ml Methylenchlorid vorgelegt. Bei Raumtemperatur tropft man eine Lösung aus 550 mg Methansulfonsäurechlorid und 5 ml Methylenchlorid zu und rührt 4 Stunden bei Raumtemperatur nach. Man verdünnt mit Wasser, trennt die organische Phase ab und dampft ein. Es wird über Kieselgel 60 (40-63 µm) mit Cyclohexan : Essigester 1:1 chromatographiert. Das erhaltene Öl wird mit Petrolther/Diisopropylether kristallisiert. Man erhält farblose Kristalle.
- Fp.:: 118°C
- DC :: (Kieselgel 60) Diisopropylether:Ethanol 3:2
- R_{f} :: 0,617
- Ausbeute:: 930 mg (63,7% der Theorie)

### Beispiel 7

### 6-Methoxy-4-[N-propyl-N-(butylsulfonylamidoethyl)]-amino-1,3,4,5-tetrahydrobenz[c,d]-indol

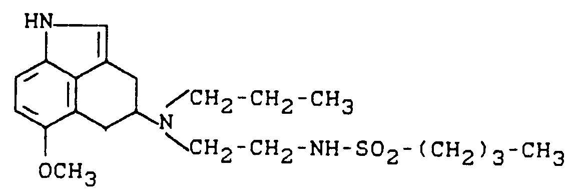

1,15 g 6-Methoxy-4-[N-propyl-N-(aminoethyl)]-amino-1,3,4,5-tetrahydrobenz[c,d]-indol und 553 mg Kaliumcarbonat werden in 40 ml Methylenchlorid vorgelegt. Bei Raumtemperatur werden 752 mg Butansulfonsäurechlorid zugetropft und 4 h bei Raumtemperatur nachgerührt. Man verdünnt mit Wasser, trennt die organische Phase ab und dampft ein. Es wird über Kieselgel 60 (40-63 µm) mit Cyclohexan : Essigester 1:1 chromatographiert. Man erhält die Verbindung als ein zähes Harz.
- DC:: (Kieselgel 60) Cyclohexan : Essigester 1:1
- R_{f} :: 0,767
- Ausbeute:: 1,24 g (76,1% der Theorie)

### Beispiel 8

### 6-Methoxy-4-[N-propyl-N-(tosylamidoethyl)]-amino-1,3,4,5-tetrahydrobenz[c,d]-indol Hydrochlorid

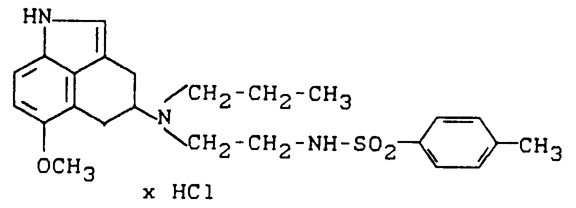

2,32 g 6-Methoxy-4-[4-propyl-N-(aminoethyl)]-amino-1,3,4,5-tetrahydrobenz[c,d]-indol werden mit 4,5 ml 50%iger Kaliumcarbonatlösung in 45 ml Methylenchlorid vorgelegt. Unter kräftigem Rühren tropft man bei Raumtemperatur eine Lösung aus 1,7 g Tosylchlorid und 15 ml Methylenchlorid zu. Man rührt 3 Stunden bei Raumtemperatur nach. Man verdünnt mit Wasser und Methylenchlorid, trennt die Phasen und dampft die organische Phase ein. Man chromatographiert über Kieselgel 60 (40 - 63 µm) mit Diisopropylether : Ethanol 3:2.
Das erhaltene Öl wird in Diethylether gelöst und mit etherischer Salzsäure versetzt. Das Lösemittel wird abgezogen und das pulverförmige Hydrochlorid im Hochvakuum bei 50°C getrocknet.
- DC :: (Kieselgel 60) Diisopropylether : Ethanol 3:2
- R_{f} :: 0,708
- Ausbeute:: 2 g (52% der Theorie)

### Beispiel 9

### 6-Methoxy-4-[N-propyl-N-(2-naphthylsulfonylamidoethyl)]-amino-1,3,4,5-tetrahydrobenz[c,d]-indol-Hydrochlorid

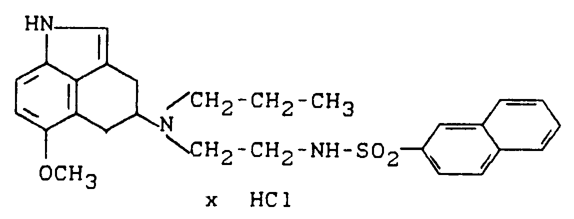

862 mg 6-Methoxy-4-[N-propyl-N-(aminoethyl)]-amino-1,3,4,5-tetrahydrobenz[c,d]-indol werden mit 1,5 ml 50%iger Kaliumcarbonatlösung in 15 ml Methylenchlorid vorgelegt. Bei Raumtemperatur wird eine Lösung aus 748 mg 2-Naphthalinsulfonsäurechlorid und 5 ml Methylenchlorid zugetropft. Man rührt über Nacht bei Raumtemperatur nach, verdünnt dann mit Wasser und Methylenchlorid und trennt die organische Phase ab. Das Rohprodukt wird über Kieselgel 60 (40 - 63 µm) mit Cyclohexan : Essigester 1:1 chromatographiert. Das Produkt wird in Essigester gelöst. Dann wird mit etherischer Salzsäure das Salz gefällt. Man verdünnt mit Ether und saugt ab. Die Verbindung wird als pulverförmiges Hydrochlorid erhalten.
- DC :: Cyclohexan : Essigester 1:1
- R_{f} :: 0,307
- Ausbeute:: 910 mg (59,1% der Theorie)

### Beispiel 10

### 6-Methoxy-4-[N-propyl-N-(1-phenylureidoethyl)]-amino-1,3,4,5-tetrahydrobenz[c,d]-indol

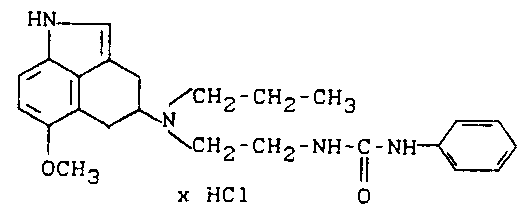

1,18 g 6-Methoxy-4-[N-propyl-N-(aminoethyl)]-amino-1,3,4,5-tetrahydrobenz[c,d]-indol werden in 20 ml Methylenchlorid vorgelegt. Man gibt einige Tropfen Triethylamin zu und tropft dann 420 mg Phenylisocyanat bei Raumtemperatur zu. Man rührt über Nacht bei Raumtemperatur nach und dampft ein. Der Rückstand wird über Kieselgel 60 (40 - 63 µm) mit Essigester : Ethanol 9:1 chromatographiert. Man löst den Rückstand in Diethylether und fällt mit etherischer Salzsäure das Salz.
Man erhält die Substanz als pulverförmiges Hydrochlorid.
- DC :: (Kieselgel 60) Essigester : Ethanol 9:1
- R_{f} :: 0,176
- Ausbeute:: 760 mg (42% der Theorie)

### Beispiel 11

### 6-Methoxy-4-[N-propyl-N-(aminoethyl)]-amino-1,3,4,5-tetrahydrobenz[c,d]-indol

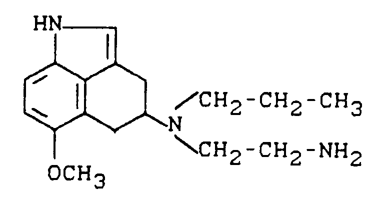

3,44 g Lithiumaluminiumhydrid werden in 50 ml absolutem Diethylether vorgelegt und zum Rückfluß erhitzt. Man tropft eine Lösung aus 6,4 g 6-Methoxy-4-[N-propyl-N-(cyanomethyl)]-amino-1,3,4,5-tetrahydrobenz[c,d]-indol in 75 ml absolutem Diethylether zu und rührt 4 Stunden unter Rückfluß nach. Dann wird vorsichtig mit Wasser zersetzt und abgesaugt. Der Rückstand wird zweimal mit Essigester ausgekocht. Man vereinigt die organischen Phasen und dampft ein. Aus dem öligen Rückstand läßt sich das Produkt mit Petrolether : Diisopropylether kristallisieren. Man erhält ein farbloses Pulver.
- Fp.:: 146 - 147°C
- Ausbeute:: 4,6 g (70,8% der Theorie)

### Beispiel 12

### 6-Methoxy-4-[N-propyl-N-(cyanomethyl)]-amino-1,3,4,5-tetrahydrobenz[c,d]-indol

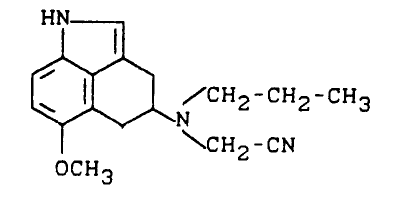

7,2 g 6-Methoxy-4-propylamino-1,3,4,5-tetrahydrobenz[c,d]-indol werden mit 11,5 g Chloracetonitril in Gegenwart von 20,7 g Kaliumcarbonat und 1 g Kaliumiodid in 150 ml Methylethylketon bei 70°C zur Reaktion gebracht. Die Reaktion ist nach 3,5 - 4 Stunden beendet. Man filtriert und dampft ein. Das Rohprodukt wird über Kieselgel 60 (63 - 200 µm) mit Cyclohexan : Essigester 1:1 chromatographiert. Man erhält die Substanz als zähes Öl.
- DC :: (Kieselgel 60) Cyclohexan : Essigester 1:1
- R_{f} :: 0.447
- Ausbeute:: 8 g (94% der Theorie)

### Beispiel 13

### 6-Methoxy-4-propylamino-1,3,4,5-tetrahydrobenz[c,d]-indol

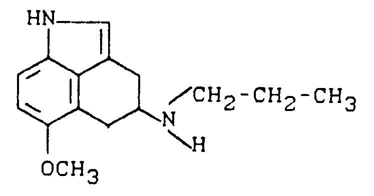

1,25 g 6-Methoxy-4-amino-1,3,4,5-tetrahydrobenz[c,d]-indol werden in 40 ml Methanol vorgelegt. Man gibt 265 µl Eisessig und 492 mg Natriumcyanoborhydrid zu und erhitzt auf 60°C. Bei dieser Temperatur tropft man 440 µl Propionaldehyd während 30 min zu. Es wird 1 h bei 60°C nachgerührt und dann eingedampft. Der Rückstand wird in Methylenchlorid aufgenommen und einmal mit Wasser gewaschen. Man dampft die organische Phase ein, nimmt den Rückstand in Essigester auf und verrührt 10 min lang mit 5 molarer Natronlauge. Man trennt die organische Phase ab, extrahiert noch einmal mit Essigester und dampft ein. Der Rückstand wird über Kieselgel 60 (40 - 63 µm) mit Diisopropylether : Ethanol 3:2 chromatographiert. Das Produkt ist ein zähes Öl.
- DC :: (Kieselgel 60) Diisopropylether : Ethanol 3:2
- R_{f} :: 0,204
- Ausbeute :: 800 mg (53% der Theorie)

### Beispiel 14

### 6-Methoxy-4-(dimethylsulfamoylethyl)-amino-1,3,4,5-tetrahydrobenz[c,d]-indol

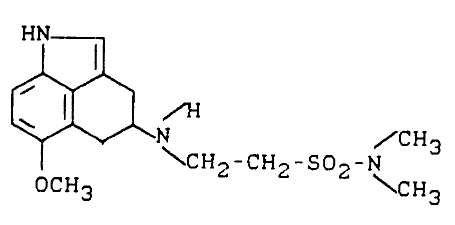

1 g 6-Methoxy-4-oxo-1,3,4,5-tetrahydrobenz[c,d]-indol werden mit 5 g Molsieb (4 Å) in 30 ml Methanol vorgelegt. Man gibt 2,3 g Essigsäure zu und kühlt auf 0°C ab. 1,14 g 2-Dimethylssulfamoylethylamin werden zugegeben und es wird 30 min bei 0°C nachgerührt. Man läßt auf Raumtemperatur kommen und gibt 1,3 g Natriumcyanoborhydrid zu und rührt 4 Stunden bei Raumtemperatur nach. Dann wird filtriert und eingedampft. Der Rückstand wird in Essigester aufgenommen, mit 20%iger Natronlauge versetzt und 15 min verrührt. Man trennt die Phasen, wäscht die wäßrige Phase mit Essigester und dampft die vereinigten organischen Phasen ein. Man chromatographiert über Kieselgel 60 (40 -6 3 µm) mit Diisopropylamin : Ethanol 4:1 als Fließmittel. Die Substanz wird als ein zähes Öl erhalten.
- DC :: (Kieselgel 60) Essigester : Ethanol 9:1
- R_{f} :: 0,189
- Ausbeute:: 400 mg (24% der Theorie)

### Beispiel 15

### 6-Methoxy-4-[N-propyl-N-(dimethylsulfamoylethyl)]-amino-1,3,4,5-tetrahydrobenz[c,d]-indol

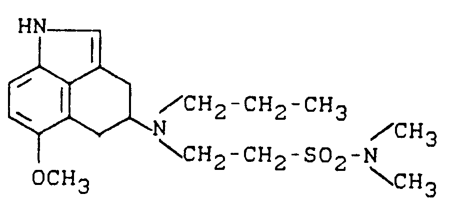

362 mg 6-Methoxy-4-(dimethylsulfamoylethyl)-amino-1,3,4,5-tetrahydrobenz[c,d]-indol werden in 7,5 ml Methanol vorgelegt. Man gibt 480 mg Eisessig und 93 mg Propionaldehyd zu. Dann werden 270 mg Natriumcyanoborhydrid zugegeben und es wird 2 h bei Raumtemperatur nachgerührt. Nach dem Eindampfen wird der Rückstand in Essigester aufgenommen, mit 20%iger Natronlauge versetzt und 10 Minuten verrührt. Man trennt die Essigesterphase ab und extrahiert sie mit 3 N Salzsäure. Die Salzsäurephase wird alkalisch gestellt und mehrfach mit Essigester extrahiert. Man dampft die organische Phase ein und chromatographiert über Kieselgel 60 (63 - 200 µm) mit Essigester. Das erhaltene Produkt wird nochmals über Kieselgel 60 (63 - 200 µm) mit einem Cyclohexan/Essigester-Gradienten unter Zusatz von wenig Triethylamin chromatographiert. Das erhaltene Produkt wird in Ether gelöst und mit etherischer Salzsäure das Hydrochlorid gefällt. Man erhält einen farblosen hygroskopischen Feststoff.
- DC :: (Kieselgel 60) Essigester
- R_{f} :: 0,466
- Ausbeute :: 125 mg (28,1% der Theorie)

### Beispiel 16

### 6-Methoxy-4-(methylsulfonylamidopropyl)-amino-1,3,4,5-tetrahydrobenz[c,d]-indol Hydrochlorid

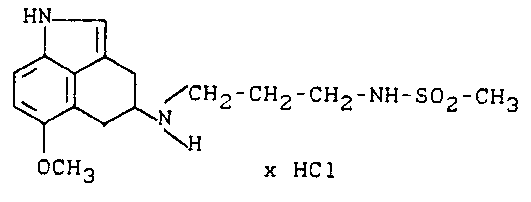

500 mg Palladium-Kohle (10%ig) werden in 20 ml Methanol und 5 ml Methanol/Salzsäure (1 mol x 1⁻¹) vorhydriert. Man gibt eine Lösung aus 1,2 g 6-Methoxy-4-[N-benzyl-N-(methylsulfonylamidopropyl)]-amino-1,3,4,5-tetrahydrobenz[c,d]-indol in 40 ml Methanol zu und hydriert bis zur beendeten Wasserstoffaufnahme. Man filtriert vom Katalysator ab, neutralisiert die Lösung und dampft ein. Der Rückstand wird über Kieselgel 60 (40 - 63 µm) mit
Essigester : Ethanol 9:1 chromatographiert. Die sauberen Fraktionen werden vereinigt, mit etherischer Salzsäure versetzt und das Lösemittel wird bei Raumtemperatur abgezogen. Man erhält die Verbindung als pulverförmiges Hydrochlorid.
- DC :: (Kieselgel 60) Diisopropylether : Ethanol 3:2
- R_{f} :: 0,274
- Ausbeute:: 557 mg (53,3% der Theorie)

### Beispiel 17

### 6-Methoxy-4-[N-benzyl-N-(methylsulfonylamidopropyl)]-amino-1,3,4,5-tetrahydrobenz[c,d]-indol

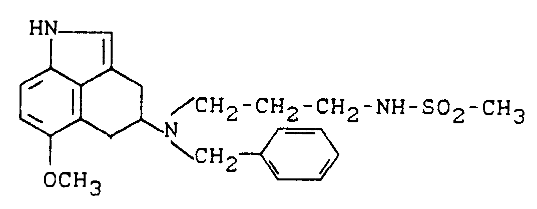

1,7 g 6-Methoxy-4-[N-benzyl-N-(aminopropyl)]-amino-1,3,4,5-tetrahydrobenz[c,d]-indol und 672 mg Kaliumcarbonat werden in 50 ml Methylenchlorid vorgelegt. Bei Raumtemperatur wird eine Lösung aus 613 mg Methansulfonsäurechlorid und 10 ml Methylenchlorid zugetropft und über Nacht bei Raumtemperatur nachgerührt. Dann wird abfiltriert und einrotiert. Der Rückstand wird über Kieselgel 60 (40 - 63 µm) mit Cyclohexan : Essigester 1:1 chromatographiert. Man erhält Kristalle vom Fp.: 132 - 133°C.
- DC :: (Kieselgel 60) Cyclohexan : Essigester 1:1
- R_{f} :: 0,182
- Ausbeute:: 1,2 g (57,7% der Theorie)

### Beispiel 18

### 6-Methoxy-4-(4-fluorphenylsulfonylamidopropyl)-amino-1,3,4,5-tetrahydrobenz[c,d]-indol Hydrochlorid

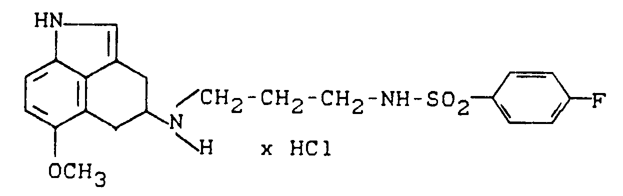

800 mg Palladium-Kohle (10%ig) werden in 25 ml Methanol und 6,5 ml Methanol/Salzsäure (1 mol x 1⁻¹) vorhydriert. Man gibt eine Lösung aus 1,6 g 6-Methoxy-4-[N-benzyl-N-(4-fluorphenylsulfonylamidopropyl)]-amino-1,3,4,5-tetrahydrobenz[c,d]-indol in 25 ml Methanol zu und hydriert bis zur beendeten Wasserstoffaufnahme. Man filtriert vom Katalysator ab, neutralisiert die Lösung und dampft ein. Der Rückstand wird über Kieselgel 60 (40 - 63 µm) mit Essigester : Ethanol 9:1 chromatographiert. Man nimmt das Produkt in Ether auf und fällt mit etherischer Salzsäure das Salz.
- DC :: (Kieselgel 60) Diisopropylether : Ethanol 3:2
- R_{f} :: 0,481
- Ausbeute:: 840 mg (58,7% der Theorie)

### Beispiel 19

### 6-Methoxy-4-[N-benzyl-N-(4-fluorphenylsulfonylamidopropyl)]-amino-1,3,4,5-tetrahydrobenz[c,d]-indol

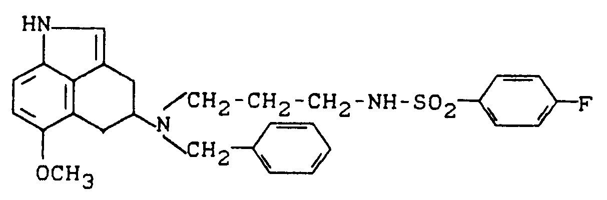

12,5 g 6-Methoxy-4-[N-benzyl-N-(aminopropyl)]-amino-1,3,4,5-tetrahydrobenz[c,d]-indol und 470 mg Kaliumcarbonat werden in 30 ml Methylenchlorid vorgelegt. Bei Raumtemperatur tropft man eine Lösung aus 730 mg 4-Fluorbenzolsulfonsäurechlorid in 5 ml Methylenchlorid zu und rührt über Nacht bei Raumtemperatur nach. Man verdünnt mit Wasser und Methylenchlorid, trennt die organische Phase ab und dampft ein. Das Rohprodukt wird mit Cyclohexan : Essigester 7:3 über Kieselgel 60 (40 - 63 µm) chromatographiert. Man erhält die Substanz als zähes Harz.
- DC :: (Kieselgel 60) Cyclohexan : Essigester 7:3
- R_{f} :: 0,172
- Ausbeute:: 12,5 g (72,5% der Theorie)

### Beispiel 20

### 6-Methoxy-4-(N-benzyl-N-aminopropyl)-amino-1,3,4,5-tetrahydrobenz[c,d]-indol

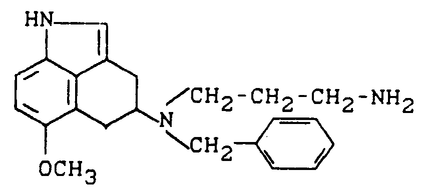

882 mg Lithiumaluminiumhydrid werden unter Argon in 15 ml absolutem Diethylether vorgelegt. Unter Rückfluß tropft man eine Suspension aus 2 g 6-Methoxy-4-[N-benzyl-N-(2-cyanoethyl)-amino-1,3,4,5-tetrahydrobenz[c,d]-indol in 50 ml absolutem Diethylether zu. Man rührt 4 Stunden unter Rückfluß nach und kühlt dann auf Raumtemperatur ab. Man zersetzt mit 8 ml Essigester, 1 ml Wasser und 2 ml 15%iger Kalilauge. Man saugt ab und wäscht den Rückstand dreimal mit Essigester nach. Die Mutterlauge wird eingedampft und der erhaltene Rückstand über Kieselgel 60 (40 - 63 µm) mit Methanol : Triethylamin 95:5 chromatographiert. Man erhält die Substanz als zähes Öl.
- DC :: (Kieselgel 60) Methanol : Triethylamin 95:5
- R_{f} :: 0,186
- Ausbeute:: 1,35 g (66,5% der Theorie)

### Beispiel 21

### 6-Methoxy-4-[N-benzyl-N-(2-cyanoethyl)]-amino-1,3,4,5-tetrahydrobenz[c,d]-indol

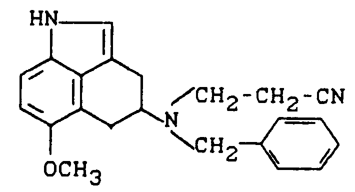

2,05 g 6-Methoxy-4-benzylamino-1,3,4,5-tetrahydrobenz[c,d]-indol und 1,86 g Acrylnitril und 25 mg Kupfer(II)acetat werden 12 Stunden unter Rückfluß gerührt. Man chromatographiert das Reaktionsgemisch mit Cyclohexan : Essigester 7:3 über Kieselgel 60 (63 - 200 µm). Man erhält ein farbloses Pulver vom Schmelzpunkt 126°C.
- DC :: (Kieselgel 60) Cyclohexan : Essigester 1:1
- R_{f} :: 0,445
- Ausbeute:: 2,05 g (84,7% der Theorie)

### Beispiel 22

### 6-Methoxy-4-benzylamino-1,3,4,5-tetrahydrobenz[c,d]-indol

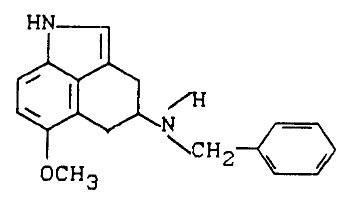

2 g 6-Methoxy-4-amino-1,3,4,5-tetrahydrobenz[c,d]-indol werden in 75 ml Methanol vorgelegt. Man gibt 450 µl Essigsäure und 800 mg Natriumcyanoborhydrid zu, erhitzt auf 60°C und tropft 1,12 g Benzaldehyd während 30 Minuten zu. Man rührt 1 Stunde bei 60°C nach und dampft dann ein. Der Rückstand wird in Essigester aufgenommen und mit 20%iger Natronlauge 10 Minuten verrührt. Man trennt die organische Phase ab und dampft ein. Der Rückstand wird in Methylenchlorid gelöst, mit A-Kohle versetzt und 1 Stunde bei Raumtemperatur gerührt. Man filtriert und dampft ein. Die erhaltenen braunen Kristalle werden über Kieselgel 60 (40 - 63 µm) mit Essigester : Ethanol 9:1 chromatographiert.
Man erhält ein Kristallpulver vom Fp.: 143 - 144°C.
- DC :: Essigester : Ethanol 9:1
- R_{f} :: 0,359
- Ausbeute:: 2,05 g (70,2% der Theorie)

### Anwendungsbeispiel

### Beispiel 23

### Affinität zu 5-HT₁-Rezeptor

In Tabelle 1 wird beispielhaft die hohe Affinität der erfindungsgemäßen Verbindungen zu 5-Hydroxytryptamin-Rezeptoren vom Subtyp 1 dargestellt. Bei den angegebenen Werten handelt es sich um Daten, die aus Rezeptorbindungsstudien mit Kalbs-Hippocampus-Membranpräparationen ermitelt wurden. Als radioaktiv markierter Ligand wurde hierzu 3H-Serotonin verwendet.

**Tabelle 1**

| Verbindung des Beispiels Nr. | Ki (nmol/l) |
|---|---|
| 1 | 2 |
| 2 | 2 |
| 5 | 1 |
| 6 | 6 |
| 8 | 1 |
| 9 | 2 |
| 15 | 8 |
| 18 | 0,7 |

Weiterhin wurden die Verbindungen auf antidepressive Wirkeigenschaften untersucht. Dazu wurde der Einfluß der Verbindungen auf das sog. "behavioral despair"-Verhalten nach Porsolt et al. Arch. Int. Pharmacodyn. Ther. 229, 327 (1977) untersucht. Beispielhaft zeigen die Verbindungen nach den Beispielen 3, 4, 7 und 13 positive Wirkung.

## Patentansprüche (Patentansprüche für folgende(n) Vertragsstaat(en): AT, BE, CH, DE, FR, GB, GR, IT, LI, LU, NL, SE)

1. 1,3,4,5-Tetrahydrobenz[c,d]indole der Formel 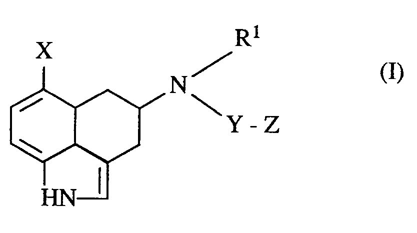 in welcher
R¹ für H, C₁-C₄-Alkyl oder Benzyl steht,
X für H, OCH₃, OH, SCH₃, F, Cl, Br, CN oder CONH₂ steht,
Y für eine geradkettige oder verzweigte, gesättigte oder ungesättigte Alkylenkette mit bis zu 6 Kohlenstoffatomen steht,
und
Z für Cyano steht, oder für eine Gruppe der Formel 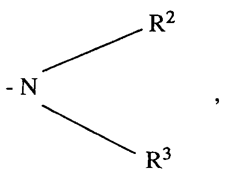 -SO₂NR⁷R⁸ oder CONR⁷R⁸ steht,
worin
R⁷ und R⁸ gleich oder verschieden sind und
für Wasserstoff, C₁-C₆-Alkyl, Phenyl, Benzyl oder Phenethyl stehen,
R² und R³ gleich oder verschieden sind und
für Wasserstoff oder C₁-C₆-Alkyl, Phenyl oder Benzyl stehen, wobei die Phenylreste durch Fluor, Chlor, Brom, C₁-C₆-Alkyl, C₁-C₆-Alkoxy oder Trifluormethyl substituiert sein können, oder
für eine Gruppe der Formel
COR⁹ oder -SO₂R¹⁰ stehen,
worin
R⁹ Wasserstoff oder eine Gruppe NHR¹¹ bedeutet, oder C₁-C₆-Alkyl oder C₁-C₆-Alkoxy bedeutet oder gegebenenfalls durch C₁-C₆-Alkyl, C₁-C₆-Alkoxy, Fluor, Chlor, Brom, Trifluormethyl, Dimethylamino oder Diethylamino substituiertes Phenyl, Benzyl, Benzyloxy, Thienyl, Furyl, Pyridyl, Pyrimidyl, Chinolyl, Isochinolyl, Benzothiazolyl, Benzoxazolyl, Thiazolyl, Oxazolyl, Isoxazolyl oder Isothiazolyl bedeutet,
R¹⁰ gegebenenfalls durch Cyano, Fluor, Chlor, Brom, Trifluormethyl oder C₁-C₆-Alkoxycarbonyl substituiertes C₁-C₆-Alkyl bedeutet, oder gegebenenfalls durch C₁-C₄-Alkyl, C₁-C₆-Alkoxy, Fluor, Chlor, Brom, Trifluormethyl, Dimethylamino oder Dimethylamino substituiertes Phenyl, Naphthyl, Benzyl, Thienyl, Furyl, Pyrimidyl, Pyridyl, Chinolyl, Isochinolyl, Benzothiazolyl, Benzoxazolyl, Thiazolyl, Oxazolyl, Isoxazolyl oder Isothiazolyl bedeutet, oder
eine Gruppe -NR⁷R⁸ bedeutet,
wobei
R⁷ und R⁸ die oben angegebene Bedeutung haben,
und
R¹¹ gegebenenfalls durch Cyano, Fluor, Chlor oder Brom substituiertes C₁-C₆-Alkyl bedeutet, oder gegebenenfalls durch C₁-C₆-Alkyl, C₁-C₆-Alkoxy, Fluor, Chlor, Brom, Trifluormethyl, Dimethylamino oder Diethylamino substituiertes Phenyl, Benzyl, Thienyl, Furyl, Pyridyl, Pyrimidyl, Chinolyl, Isochinolyl, Benzothiazolyl, Benzoxazolyl, Thiazolyl, Oxazolyl, Isoxazolyl oder Isothiazolyl bedeutet,
oder
R² und R³ zusammen mit dem Stickstoffatom einen Heterocyclus der Reihe 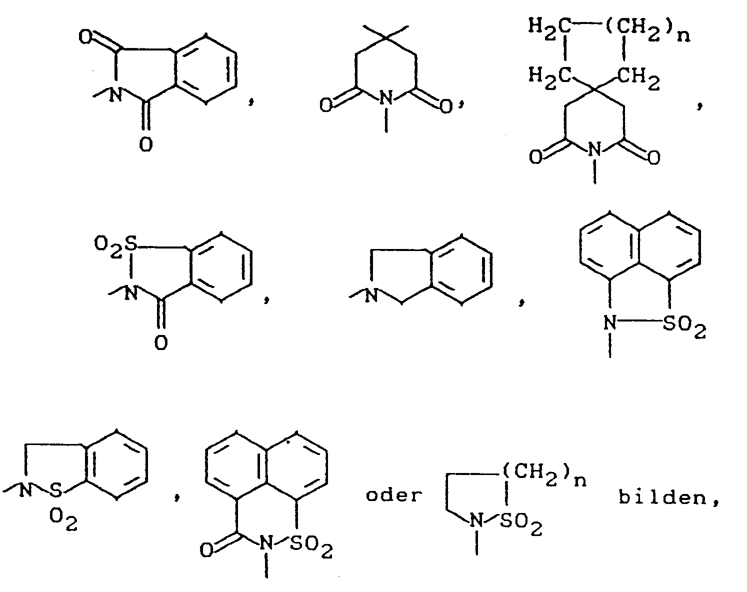 worin
n eine Zahl 1 oder 2 bedeutet
und deren Salze.

2. 1,3,4,5-Tetrahydrobenz[c,d]indole nach Anspruch 1, worin
R¹ für Wasserstoff, Methyl, Ethyl, n-Propyl oder Benzyl steht,
X für H, OCH₃, OH, SCH₃, F, Cl, CN, CONH₂ steht,
Y für eine geradkettige Alkylenkette mit 2 bis 4 Kohlenstoffatomen steht
und
Z für Cyano steht, oder für eine Gruppe der Formel 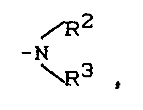 SO₂NR⁷R⁸, CONR⁷R⁸ steht,
wobei
R⁷ und R⁸ gleich oder verschieden sind und
für Wasserstoff, Methyl, Ethyl, Propyl, Isopropyl, Butyl, Isobutyl oder tert.Butyl stehen,
R² und R³ gleich oder verschieden sind, und
für Wasserstoff, Methyl, Ethyl, Propyl, Isopropyl, Butyl, Isobutyl, tert.Butyl oder
für gegebenenfalls durch Fluor, Chlor, Methyl oder Methoxy subsituiertes Phenyl, oder
für eine Gruppe -COR⁹ oder -SO₂R¹⁰ stehen,
worin
R⁹ Wasserstoff oder eine Gruppe NHR¹¹ bedeutet, oder Methyl, Ethyl, Propyl, Isopropyl, Methoxy, Ethoxy, Propoxy, Isopropoxy, oder gegebenenfalls durch Methyl, Methoxy, Fluor oder Chlor substituiertes Phenyl, Benzyl, Benzyloxy, Thienyl, Furyl, Pyridyl, Pyrimidyl, Chinolyl oder Isochinolyl bedeutet,
R¹⁰ gegebenenfalls durch Fluor, Chlor, Methoxycarbonyl, Ethoxycarbonyl, Propoxycarbonyl, Isopropoxycarbonyl, Butoxycarbonyl oder Isobutoxycarbonyl substituiertes Methyl, Ethyl, Propyl, Isopropyl, Butyl oder Isobutyl bedeutet, oder gegebenenfalls durch Methyl, Ethyl, Propyl, Isopropyl, Methoxy, Fluor oder Chlor substituiertes Phenyl, Naphthyl, Benzyl Thienyl, Furyl, Pyridyl, Pyrimidyl, Chinolyl oder Isochinolyl bedeutet, oder
eine Gruppe NR⁷R⁸ bedeutet,
wobei
R⁷ und R⁸ die oben angegebene Bedeutung haben,
und
R¹¹ gegebenenfalls durch Fluor oder Chlor substituiertes Methyl, Ethyl, Propyl, Isopropyl, Butyl, Isobutyl, Pentyl, Isopentyl, Hexyl oder Isohexyl bedeutet, oder Phenyl oder Benzyl bedeutet, das durch Fluor, Chlor, Methyl oder Methoxy substituiert sein kann,
oder
R² und R³ gemeinsam mit dem Stickstoffatom einen Heterocyclus der Reihe 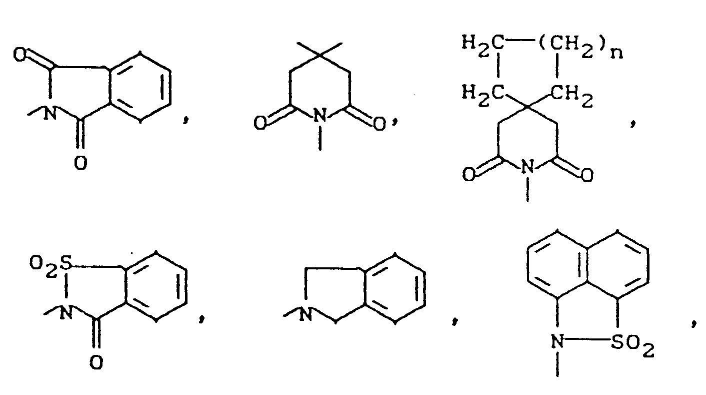 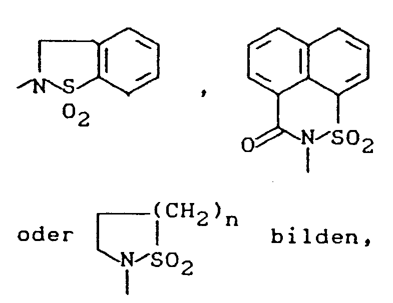 worin
n eine Zahl 1 oder 2 bedeutet
und deren Salze.

3. 1,3,4,5-Tetrahydrobenz[c,d]indole nach Anspruch 1 zur therapeutischen Behandlung.

4. Verfahren zur Herstellung von 1,3,4,5-Tetrahydrobenz[c,d]indole nach Anspruch 1
dadurch gekennzeichnet, daß man Ketone der Formel (II) 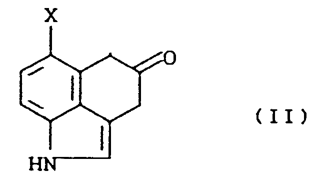 in der X die in Anspruch 1 anegebene Bedeutung besitzt,
mit Aminen der Formel (III), (IV) oder (V) 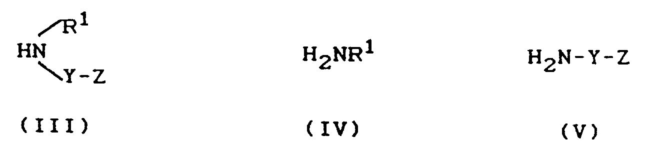 in denen R¹, Y und Z die in Anspruch 1 angegebene Bedeutung haben, reduktiv aminiert [A].

5. Verfahren zur Herstellung von 1,3,4,5-Tetrahydrobenz[c,d]indolen nach Anspruch 1
dadurch gekennzeichnet, daß man Verbindungen der allgemeinen Formel (VI) 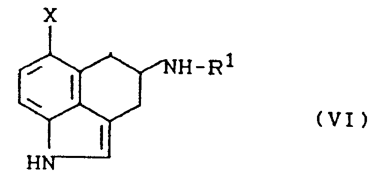 in der R¹ und X die in Anspruch 1 angegebene Bedeutung haben,
mit Alkylierungsmitteln der Formel (VII)
L-Y-Z (VII)
in der Y und Z die in Anspruch 1 angegebene Bedeutung haben, und L eine "leaving group" für Alkylierungsmittel bedeutet, umsetzt [B],
oder mit Aldehyden der Formel (VIII)
OCH-Y¹-Z (VIII)
in denen Z die in Anspruch 1 angegebene Bedeutung besitzt und Y¹ eine um eine Methylengruppe Verkürzte Alkylenkette Y ist, reduktiv alkyliert [C],
oder mit reaktiven Säurederivaten der allgemeinen Formel (IX)
M-OC-Y¹-Z (IX)
in der Y¹ und Z die oben angegebene Bedeutung haben und M eine in Acylierungsmitteln übliche leaving group wie Chlor, Brom, Alkoxy, Aryloxy, Imidazolyl, Thiazolyl, Methansulfonyloxy oder Alkoxycarbonyloxy bedeutet, umsetzt und die erhaltenen Säureamide katalytisch mit Wasserstoffperoxid oder mit komplexen Metallhydriden zu Verbindungen der Formel (I) reduziert [D].

6. Verfahren zur Herstellung von 1,3,4,5-Tetrahydrobenz[c,d]indole nach Anspruch 1
dadurch gekennzeichnet, daß man Verbindungen der Formel (X) 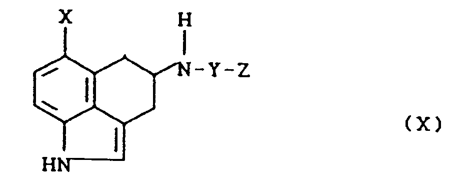 in der X, Y und Z die in Anspruch 1 angegebene Bedeutung haben,
mit Alkylierungsmitteln der Formel (XI)
R¹-L (XI)
in der R¹ und L die in Anspruch 1 angegebene Bedeutung haben, alkyliert,
oder mit Aldehyden der Formel (XII)
R¹³-CHO (XII)
in der R¹³ ein um eine Methylengruppe verkürzter Rest R¹ ist, reduktiv alkyliert
oder mit reaktiven Säurederivaten der allgemeinen Formel (XIII)
M-CO-R¹³ (XIII)
in der R¹³ die oben angegebene Bedeutung und M die in Anspruch 5 angegebene Bedeutung haben,
umsetzt und die erhaltenen Säureamide katalytisch mit Wasserstoffperoxid oder mit komplexen Metallhydriden zu Verbindungen der Formel (I) reduziert.

7. Arzneimittel, enthaltend 1,3,4,5-Tetrahydrobenz[c,d]indole nach Anspruch 1.

8. Arzneimittel nach Anspruch 7, dadurch gekennzeichnet, daß es 0,5 bis 90 Gew.-% an 1,3,4,5-Tetrahydrobenz[c,d]indole, bezogen auf die Gesamtmischung enthält.

9. Verwendung von 1,3,4,5-Tetrahydrobenz[c,d]indole nach Anspruch 1, zur Herstellung von Arzneimitteln.

10. Verwendung nach Anspruch 9, zur Herstellung von Arzneimitteln zur Behandlung von Erkrankung des zentralen Nervensystems.

## Patentansprüche (Patentansprüche für folgende(n) Vertragsstaat(en): ES)

1. Verfahren zur Herstellung von 1,3,4,5-Tetrahydrobenz[c,d]indolen der Formel 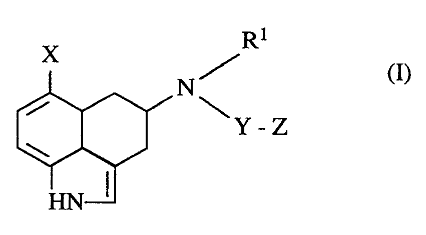 in welcher
R¹ für H, C₁-C₄-Alkyl oder Benzyl steht,
X für H, OCH₃, OH, SCH₃, F, Cl, Br, CN oder CONH₂ steht,
Y für eine geradkettige oder Verzweigte, gesättigte oder ungesättigte Alkylenkette mit bis zu 6 Kohlenstoffatomen steht,
und
Z für Cyano steht, oder für eine Gruppe der Formel 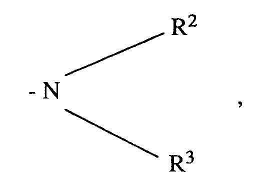 -SO₂NR⁷R⁸ oder CONR⁷R⁸ steht,
worin
R⁷ und R⁸ gleich oder verschieden sind und
für Wasserstoff, C₁-C₆-Alkyl, Phenyl, Benzyl oder Phenethyl stehen,
R² und R³ gleich oder verschieden sind und
für Wasserstoff oder C₁-C₄ Alkyl, Phenyl oder Benzyl stehen, wobei die Phenylreste durch Fluor, Chlor, Brom, C₁-C₆-Alkyl, C₁-C₆-Alkoxy oder Trifluormethyl substituiert sein können, oder
für eine Gruppe der Formel
COR⁹ oder -SO₂R¹⁰ stehen,
worin
R⁹ Wasserstoff oder eine Gruppe NHR¹¹ bedeutet, oder C₁-C₆-Alkyl oder C₁-C₆-Alkoxy bedeutet, oder gegebenenfalls durch C₁-C₆-Alkyl, C₁-C₆-Alkoxy, Fluor, Chlor, Brom, Trifluormethyl, Dimethylamino oder Diethylamino substituiertes Phenyl, Benzyl, Benzyloxy, Thienyl, Furyl, Pyridyl, Pyrimidyl, Chinolyl, Isochinolyl, Benzothiazolyl, Benzoxazolyl, Thiazolyl, Oxazolyl, Isoxazolyl oder Isothiazolyl bedeutet,
R¹⁰ gegebenenfalls durch Cyano, Fluor, Chlor, Brom, Trifluormethyl oder C₁-C₆-Alkoxycarbonyl substituiertes C₁-C₆-Alkyl bedeutet, oder gegebenenfalls durch C₁-C₄-Alkyl, C₁-C₆-Alkoxy, Fluor, Chlor, Brom, Trifluormethyl, Dimethylamino oder Dimethylamino substituiertes Phenyl, Naphthyl, Benzyl, Thienyl, Furyl, Pyrimidyl, Pyridyl, Chinolyl, Isochinolyl, Benzothiazolyl, Benzoxazolyl, Thiazolyl, Oxazolyl, Isoxazolyl oder Isothiazolyl bedeutet, oder
eine Gruppe -NR⁷R⁸ bedeutet,
wobei R⁷ und R⁸ die oben angegebene Bedeutung haben,
und
R¹¹ gegebenenfalls durch Cyano, Fluor, Chlor oder Brom substituiertes C₁-C₆-Alkyl bedeutet, oder gegebenenfalls durch C₁-C₆-Alkyl, C₁-C₆-Alkoxy, Fluor, Chlor, Brom, Trifluomethyl, Dimethylamino oder Diethylamino substituiertes Phenyl, Benzyl, Thienyl, Furyl, Pyridyl, Pyrimidyl, Chinolyl, Isochinolyl, Benzothiazolyl, Benzoxazolyl, Thiazolyl, Oxazolyl, Isoxazolyl oder Isothiazolyl bedeutet,
oder
R² und R³ zusammen mit dem Stickstoffatom einen Heterocyclus der Reihe 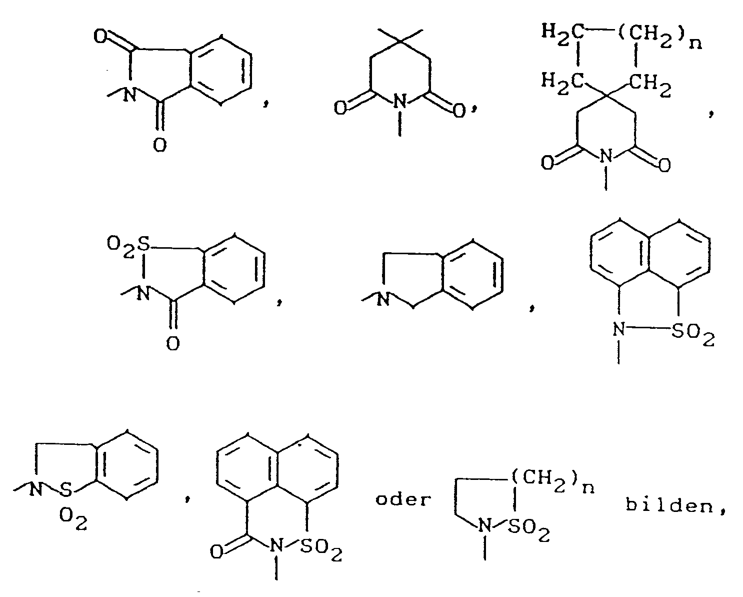 worin
n eine Zahl 1 oder 2 bedeutet
und deren Salze,
dadurch gekennzeichnet, daß man Ketone der Formel (II) 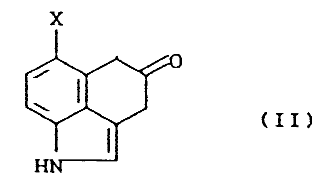 in der X die oben angegebene Bedeutung besitzt,
mit Aminen der Formel (III), (IV) oder (V) 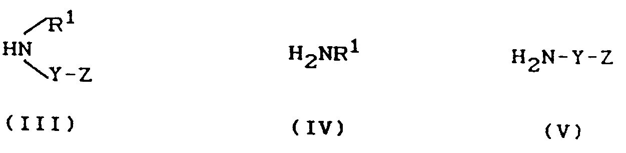 in denen R¹, Y und Z die oben angegebene Bedeutung haben, reduktiv aminiert [A].

2. Verfahren zur Herstellung von 1,3,4,5-Tetrahydrobenz[c,d]indolen nach Anspruch 1
dadurch gekennzeichnet, daß man Verbindungen der allgemeinen Formel (VI) 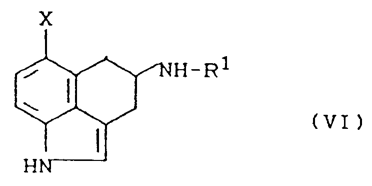 in der R¹ und X die in Anspruch 1 angegebene Bedeutung haben,
mit Alkylierungsmitteln der Formel (VII)
L-Y-Z (VII)
in der Y und Z die in Anspruch 1 angegebene Bedeutung haben, und L eine "leaving group" für Alkylierungsmittel bedeutet, umsetzt [B],
oder mit Aldehyden der Formel (VIII)
OCH-Y¹-Z (VIII)
in denen Z die in Anspruch 1 angegebene Bedeutung besitzt und Y¹ eine um eine Methylengruppe verkürzte Alkylenkette Y ist, reduktiv alkyliert [C],
oder mit reaktiven Säurederivaten der allgemeinen Formel (IX)
M-OC-Y¹-Z (IX)
in der Y¹ und Z die oben angegebene Bedeutung haben und M eine in Acylierungsmitteln übliche leaving group wie Chlor, Brom, Alkoxy, Aryloxy, Imidazolyl, Thiazolyl, Methansulfonyloxy oder Alkoxycarbonyloxy bedeutet, umsetzt und die erhaltenen Säureamide katalytisch mit Wasserstoffperoxid oder mit komplexen Metallhydriden zu Verbindungen der Formel (I) reduziert [D].

3. Verfahren zur Herstellung von 1,3,4,5-Tetrahydrobenz[c,d]indole nach Anspruch 1
dadurch gekennzeichnet, daß man Verbindungen der Formel (X) 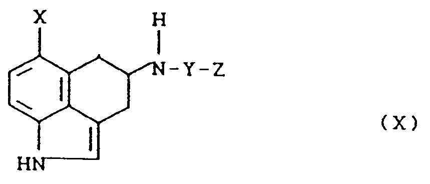 in der X, Y und Z die in Anspruch 1 angegebene Bedeutung haben,
mit Alkylierungsmitteln der Formel (XI)
R¹-L (XI)
in der R¹ und L die in Anspruch 2 angegebene Bedeutung haben, alkyliert,
oder mit Aldehyden der Formel (XII)
R¹³-CHO (XII)
in der R¹³ ein um eine Methylengruppe verkürzter Rest R¹ ist, reduktiv alkyliert
oder mit reaktiven Säurederivaten der allgemeinen Formel (XIII)
M-CO-R¹³ (XIII)
in der R¹³ die oben angegebene Bedeutung und M die in Anspruch 2 angegebene Bedeutung haben,
umsetzt und die erhaltenen Säureamide katalytisch mit Wasserstoffperoxid oder mit komplexen Metallhydriden zu Verbindungen der Formel (I) reduziert.

4. Verfahren nach Anspruch 1, dadurch gekennzeichnet, daß man die reduktive Aminierung mit komplexen Borhydriden oder Aluminiumhydriden durchführt.

## Claims (Claims for the following Contracting State(s): AT, BE, CH, DE, FR, GB, GR, IT, LI, LU, NL, SE)

1. 1,3,4,5-Tetrahydrobenz[c,d]indoles of the formula 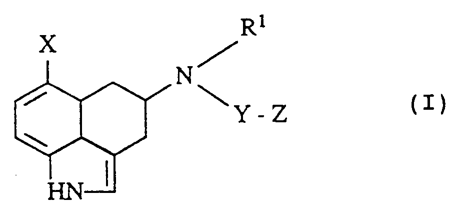 in which
R¹ represents H, C₁-C₄-alkyl or benzyl,
X represents H, OCH₃, OH, SCH₃, F, Cl, Br, CN or CONH₂,
Y represents a straight-chain or branched, saturated or unsaturated alkylene chain having up to 6 carbon atoms
and
Z represents cyano, or a group of the formula 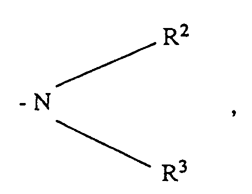 -SO₂NR⁷R⁸ oder CONR⁷R⁸ steht,
wherein
R⁷ and R⁸ are identical or different, and
represent hydrogen, C₁-C₆-alkyl, phenyl, benzyl or phenethyl,
R² and R³ are identical or different and
represent hydrogen or C₁-C₆-alkyl, phenyl or benzyl, where the phenyl radicals can be substituted by fluorine, chlorine, bromine, C₁-C₆-alkyl, C₁-C₆-alkoxy or trifluoromethyl, or
represent a group of the formula -COR⁹ or -SO₂R¹⁰.
wherein
R⁹ denotes hydrogen or a group NHR¹¹, or denotes C₁-C₆-alkyl or C₁-C₆-alkoxy, or denotes phenyl, benzyl, benzoyloxy, thienyl, furyl, pyridyl, pyrimidyl, quinolyl, isoquinolyl, bensothiazolyl, bensoxazolyl, thiazolyl, oxazolyl, isoxazolyl or isothiazolyl, each of which is optionally substituted by C₁-C₆-alkyl, C₁-C₆-alkoxy, fluorine, chlorine, bromine, trifluoromethyl, dimethylamino or diethylamino,
R¹⁰ denotes C₁-C₆-alkyl which is optionally substituted by cyano, fluorine, chlorine, bromine, trifluoromethyl or C₁-C₆-alkoxycarbonyl, or denotes phenyl, naphthyl, benzyl, thienyl, furyl, pyrimidyl, pyridyl, quinolyl, isoquinolyl, benzothiazolyl, benzoxazolyl, thiazolyl, oxazolyl, isoxazolyl or isothiazolyl, each of which is optionally substituted by C₁-C₄-alkyl, C₁-C₆-alkoxy, fluorine, chlorine, bromine, trifluoromethyl, dimethylamino or diethylamino, or
denotes a group -NR⁷R⁸,
where
R⁷ and R⁸ have the abovementioned meaning,
and
R¹¹ denotes C₁-C₆-alkyl which is optionally substituted by cyano, fluorine, chlorine or bromine, or denotes phenyl, benzyl, thienyl, furyl, pyridyl, pyrimidyl, quinolyl, isoquinolyl, benzothiazolyl, benzoxazolyl, thiazolyl, oxazolyl, isoxazolyl or isothiazolyl, each of which is optionally substituted by C₁-C₆-alkyl, C₁-C₆-alkoxy, fluorine, chlorine, bromine, trifluoromethyl, dimethylamino or diethylamino,
or
R² and R³, together with the nitrogen atom, form a heterocyclic ring from the series comprising 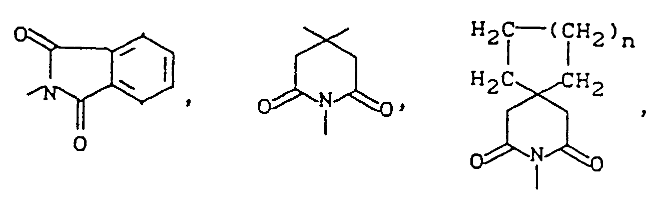 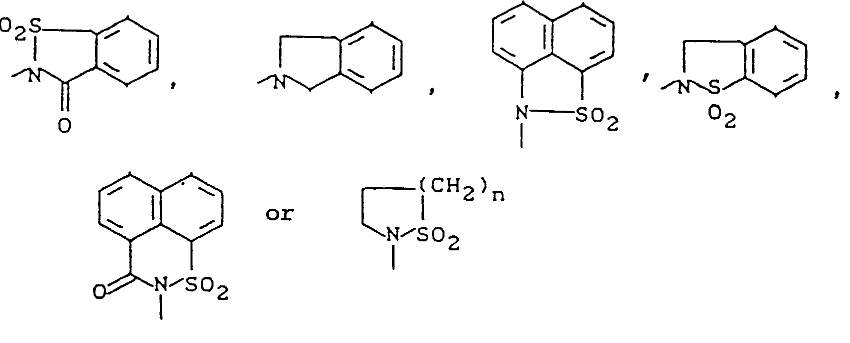 wherein
n denotes a number 1 or 2
and their salts.

2. 1,3,4,5-Tetrahydrobenz[c,d]indoles according to Claim 1
wherein
R¹ represents hydrogen, methyl, ethyl, n-propyl or benzyl,
X represents H, OCH₃, OH, SCH₃, F, Cl, CN or CONH₂,
Y represents a straight-chain alkylene chain having 2 to 4 carbon atoms
and
Z represents cyano, or a group of the formula 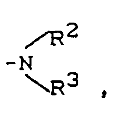 SO₂NR⁷R⁸, CONR⁷R⁸
wherein
R⁷ and R⁸ are identical or different and
represent hydrogen, methyl, ethyl, propyl, isopropyl, butyl, isobutyl or tert.butyl,
R² and R³ are identical or different, and
represent hydrogen, methyl, ethyl, propyl, isopropyl, butyl, isobutyl, tert.butyl or
represent phenyl which is optionally substituted by fluorine, chlorine, methyl or methoxy, or
represent a group -COR⁹ or -SO₂R¹⁰,
wherein
R⁹ denotes hydrogen or a group NHR¹¹ or denotes methyl, ethyl, propyl, isopropyl, methoxy, ethoxy, propoxy, isopropoxy, or phenyl, benzyl, benzyloxy, thienyl, furyl, pyridyl, pyrimidyl, quinolyl or isoquinolyl, each of which is optionally substituted by methyl, methoxy, fluorine or chlorine,
R¹⁰ denotes methyl, ethyl, propyl, isopropyl, butyl or isobutyl, each of which is optionally substituted by fluorine, chlorine, methoxycarbonyl, ethoxycarbonyl, propoxycarbonyl, isopropoxycarbonyl, butoxycarbonyl or isobutoxycarbonyl, or denotes phenyl, naphthyl, benzyl, thienyl, furyl, pyridyl, pyrimidyl, quinolyl or isoquinolyl, each of which is optionally substituted by methyl, ethyl, propyl, isopropyl, methoxy, fluorine or chlorine, or
denotes a group NR⁷R⁸,
where
R⁷ and R⁸ have the abovementioned meaning,
and
R¹¹ denotes methyl, ethyl, propyl, isopropyl, butyl, isobutyl, pentyl, isopentyl, hexyl or isohexyl, each of which is optionally substituted by fluorine or chlorine, or denotes phenyl or benzyl, each of which can be substituted by fluorine, chlorine, methyl or methoxy,
or
R² and R³, together with the nitrogen atom, form a heterocyclic ring from the series comprising 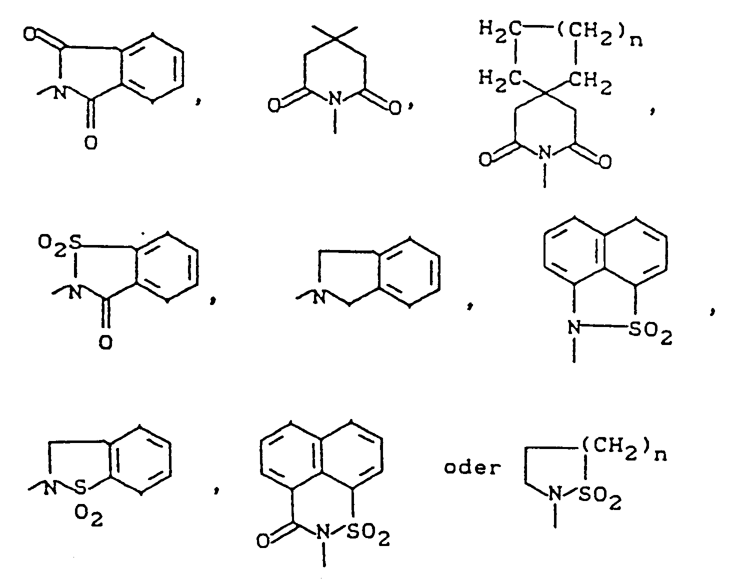 wherein
n denotes a number 1 or 2
and their salts.

3. 1,3,4,5-Tetrahydrobenz[c,d]indoles according to Claim 1 for therapeutic treatment.

4. Process for the preparation of 1,3,4,5-tetrahydrobenz[c,d]indoles according to Claim 1,
characterised in that ketones of the formula (II) 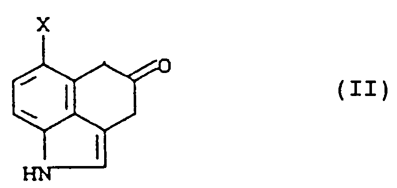 in which X has the meaning given in Claim 1,
are reductively aminated [A] with amines of the formula (III), (IV) or (V) 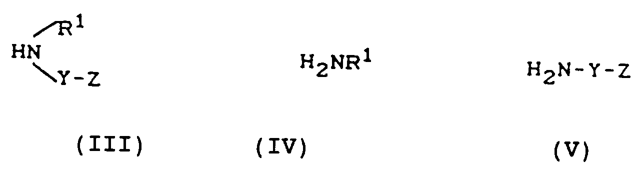 in which R¹, Y and Z have the meaning given in Claim 1.

5. Process for the preparation of 1,3,4,5-tetrahydrobenz[c,d]indoles according to Claim 1,
characterised in that compounds of the general formula (VI) 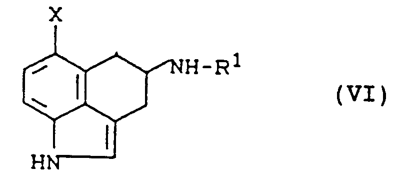 in which R¹ and X have the meaning given in Claim 1,
are reacted [B] with alkylating agents of the formula (VII)
L-Y-Z (VII)
in which Y and Z have the meaning given in Claim 1, and L denotes a leaving group customary in alkylating agents,
or reductively alkylated [C] with aldehydes of the formula (VIII)
OCH-Y¹-Z (VIII)
in which Z has the meaning given in Claim 1, and Y¹ is an alkylene chain Y shortened by one methylene group,
or reacted with reactive acid derivatives of the general formula (IX)
M-OC-Y¹-Z (IX)
in which Y¹ and Z have the abovementioned meaning and M denotes a leaving group customary in acylating agents, such as chlorine, bromine, alkoxy, aryloxy, imidazolyl, thiazolyl, methanesulphonyloxy or alkoxycarbonyloxy, and the acid amides obtained are reduced [D] catalytically with hydrogen or with complex metal hydrides to give compounds of the formula (I).

6. Process for the preparation of 1,3,4,5-tetrahydrobenz[c,d]indoles according to Claim 1,
characterised in that compounds of the formula (X) 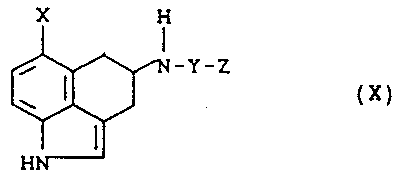 in which X, Y and Z have the meaning given in Claim 1, are alkylated with alkylating agents of the formula (XI)
R¹-L (XI)
in which R¹ and L have the meaning given in Claim 1,
or reductively alkylated using aldehydes of the formula (XII)
R¹³-CHO (XII)
in which R¹³ is a radical R¹ shortened by one methylene group,
or reacted with reactive acid derivatives of the general formula (XIII)
M-CO-R¹³ (XIII)
in which R¹³ has the abovementioned meaning and M has the meaning given in Claim 5
and the acid amides obtained are reduced catalytically with hydrogen or with complex metal hydrides to give compounds of the formula (I).

7. Medicament, containing 1,3,4,5-tetrahydrobenz[c,d]indoles according to Claim 1.

8. Medicament according to Claim 7, characterised in that it contains 0.5 to 90% by weight of 1,3,4,5-tetrahydrobenz[c,d]indoles, based on the total mixture.

9. Use of 1,3,4,5-tetrahydrobenz[c,d]indoles according to Claim 1 for the preparation of medicaments.

10. Use according to Claim 9, for the preparation of medicaments for the treatment of disorders of the central nervous system.

## Claims (Claims for the following Contracting State(s): ES)

1. Process for the preparation of 1,3,4,5-tetrahydrobenz[c,d]indoles of the formula 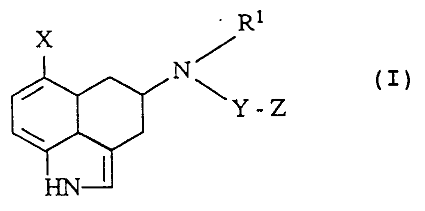 in which
R¹ represents H, C₁-C₄-alkyl or benzyl,
X represents H, OCH₃, OH, SCH₃, F, Cl, Br, CN or CONH₂,
Y represents a straight-chain or branched, saturated or unsaturated alkylene chain having up to 6 carbon atoms
and
Z represents cyano, or a group of the formula 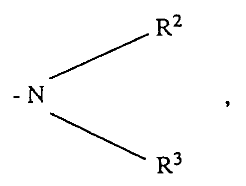 -SO₂NR⁷R⁸ oder CONR⁷R⁸
wherein
R⁷ and R⁸ are identical or different, and
represent hydrogen, C₁-C₆-alkyl, phenyl, benzyl or phenethyl,
R² and R³ are identical or different and
represent hydrogen or C₁-C₆-alkyl, phenyl or benzyl, where the phenyl radicals can be substituted by fluorine, chlorine, bromine, C₁-C₆-alkyl, C₁-C₆-alkoxy or trifluoromethyl, or
represent a group of the formula -COR⁹ or-SO₂R¹⁰.
wherein
R⁹ denotes hydrogen or a group NHR¹¹, or denotes C₁-C₆-alkyl or C₁-C₆-alkoxy, or denotes phenyl, benzyl, benzoyloxy, thienyl, furyl, pyridyl, pyrimidyl, quinolyl, isoquinolyl, benzothiazolyl, bensoxazolyl, thiazolyl, oxazolyl, isoxazolyl or isothiazolyl, each of which is optionally substituted by C₁-C₆-alkyl, C₁-C₆-alkoxy, fluorine, chlorine, bromine, trifluoromethyl, dimethylamino or diethylamino,
R¹⁰ denotes C₁-C₆-alkyl which is optionally substituted by cyano, fluorine, chlorine, bromine, trifluoromethyl or C₁-C₆-alkoxycarbonyl, or denotes phenyl, naphthyl, benzyl, thienyl, furyl, pyrimidyl, pyridyl, quinolyl, isoquinolyl, bensothiazolyl, benzoxasolyl, thiazolyl, oxazolyl, isoxazolyl or isothiazolyl, each of which is optionally substituted by C₁-C₄-alkyl, C₁-C₆-alkoxy, fluorine, chlorine, bromine, trifluoromethyl, dimethylamino or diethylamino, or
denotes a group -NR⁷R⁸,
where
R⁷ and R⁸ have the abovementioned meaning,
and
R¹¹ denotes C₁-C₆-alkyl which is optionally substituted by cyano, fluorine, chlorine or bromine, or denotes phenyl, benzyl, thienyl, furyl, pyridyl, pyrimidyl, quinolyl, isoquinolyl, benzothiazolyl, benzoxazolyl, thiazolyl, oxazolyl, isoxasolyl or isothiazolyl, each of which is optionally substituted by C₁-C₆-alkyl, C₁-C₆-alkoxy, fluorine, chlorine, bromine, trifluoromethyl, dimethylamino or diethylamino,
or
R² and R³, together with the nitrogen atom, form a heterocyclic ring from the series comprising 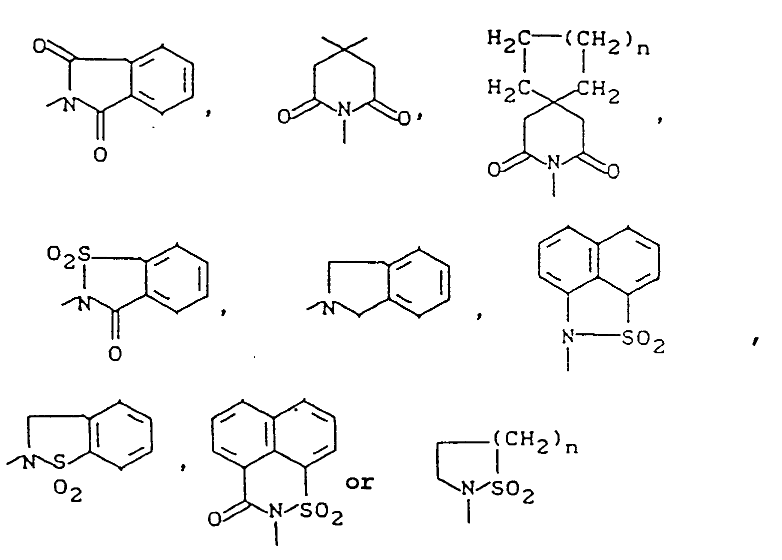 wherein
n denotes a number 1 or 2
and their salts,
characterised in that ketones of the formula (II) 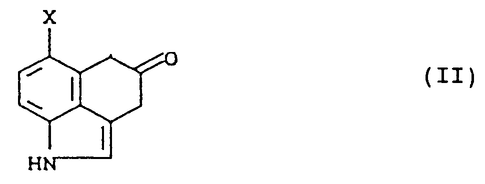 in which X has the abovementioned meaning,
are reductively aminated [A] with amines of the formula (III), (IV) or (V) 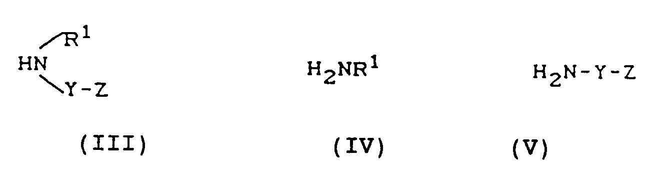 in which R¹, Y and Z have the abovementioned meaning.

2. Process for the preparation of 1,3,4,5-tetrahydrobenz[c,d]indoles according to Claim 1,
characterised in that compounds of the general formula (VI) 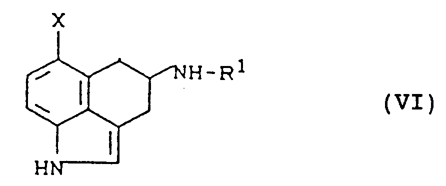 in which R¹ and X have the meaning given in Claim 1,
are reacted [B] with alkylating agents of the formula (VII)
L-Y-Z (VII)
in which Y and Z have the meaning given in Claim 1, and L denotes a leaving group customary in alkylating agents,
or reductively alkylated [C] with aldehydes of the formula (VIII)
OCH-Y¹-Z (VIII)
in which Z has the meaning given in Claim 1 and Y¹ is an alkylene chain Y shortened by one methylene group,
or reacted with reactive acid derivatives of the general formula (IX)
M-OC-Y¹-Z (IX
)
in which Y¹ and Z have the abovementioned meaning and M denotes a leaving group customary in acylating agents, such as chlorine, bromine, alkoxy, aryloxy, imidazolyl, thiazolyl, methanesulphonyloxy or alkoxycarbonyloxy, and the acid amides obtained are reduced [D] catalytically with hydrogen or with complex metal hydrides to give compounds of the formula (I).

3. Process for the preparation of 1,3,4,5-tetrahydrobenz[c,d]indoles according to Claim 1,
characterised in that compounds of the formula (X) 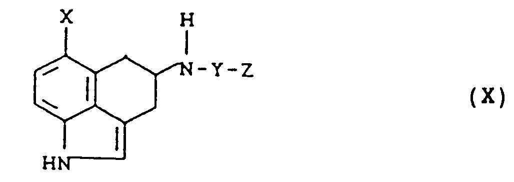 in which X, Y and Z have the meaning given in Claim 1,
are alkylated with alkylating agents of the formula (XI)
R¹-L (XI)
in which R¹ and L have the meaning given in Claim 2,
or reductively alkylated using aldehydes of the formula (XII)
R¹³-CHO (XII)
in which R¹³ is a radical R¹ shortened by one methylene group,
or reacted with reactive acid derivatives of the general formula (XIII)
M-CO-R¹³ (XIII)
in which R¹³ has the abovementioned meaning and M has the meaning given in Claim 2,
and the acid amides obtained are reduced catalytically with hydrogen or with complex metal hydrides to give compounds of the formula (I).

4. Process according to Claim 1, characterised in that the reductive amination is carried out using complex borohydrides or aluminium hydrides.

## Revendications (Revendications pour l'(les) Etat(s) contractant(s) suivant(s): AT, BE, CH, DE, FR, GB, GR, IT, LI, LU, NL, SE)

1. 1,3,4,5-tétrahydrobenz[c,d]indoles de formule 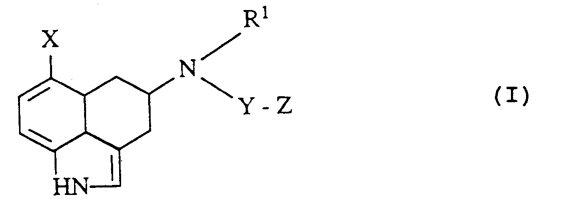 dans laquelle
R¹ représente H, un groupe alkyle en C₁ à C₄ ou benzyle,
X représente H, un groupe OCH₃, OH, SCH₃, F, Cl, Br, CN ou CONH₂,
Y est une chaîne alkylène droite ou ramifiée, saturée ou non saturée, ayant jusqu'à 6 atomes de carbone,
et
Z est un groupe cyano, ou un groupe de formule 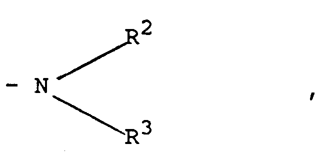 SO₂NR⁷R⁸ ou CONR⁷R⁸,
où
R⁷ et R⁸ sont identiques ou différents et représentent
l'hydrogène, un groupe alkyle en C₁ à C₆, phényle, benzyle ou phénéthyle
R² et R³ sont identiques ou différents et représentent
l'hydrogène ou un groupe alkyle en C₁ à C₆, phényle ou benzyle, les restes phényle pouvant être substitués par du fluor, du chlore, du brome, un groupe alkyle en C₁ à C₆, alkoxy en C₁ à C₆ ou trifluorométhyle, ou bien
un groupe de formule
COR⁹ ou -SO₂R¹⁰,
où
R⁹ est l'hydrogène ou un groupe NHR¹¹ ou bien un groupe alkyle en C₁ à C₆ ou alkoxy en C₁ à C₆, ou bien un groupe phényle, benzyle, benzyloxy, thiényle, furyle, pyridyle, pyrimidyle, quinolyle, isoquinolyle, benzothiazolyle, benzoxazolyle, thiazolyle, oxazolyle, isoxazolyle ou isothiazolyle portant éventuellement un substituant alkyle en C₁ à C₆, alkoxy en C₁ à C₆, fluoro, chloro, bromo, trifluorométhyle, diméthylamino ou diéthylamino,
R¹⁰ désigne un groupe alkyle en C₁ à C₆ portant facultativement un substituant cyano, fluoro, chloro, bromo, trifluorométhyle ou (alkoxy en C₁ à C₆)carbonyle, ou bien un groupe phényle, naphtyle, benzyle, thiényle, furyle, pyrimidyle, pyridyle, quinolyle, isoquinolyle, benzothiazolyle, benzoxazolyle, thiazolyle, oxazolyle, isoxazolyle ou isothiazolyle portant facultativement un substituant alkyle en C₁ à C₄, alkoxy en C₁ à C₄, fluoro, chloro, bromo, trifluorométhyle, diéthylamino ou diméthylamino, ou bien
un groupe -NR⁷R⁸,
dans lequel
R⁷ et R⁸ ont la définition indiquée ci-dessus et
R¹¹ représente un groupe alkyle en C₁ à C₆ portant éventuellement un substituant cyano, fluoro, chloro ou bromo, ou un groupe phényle, benzyle, thiényle, furyle, pyridyle, pyrimidyle, quinolyle, isoquinolyle, benzothiazolyle, benzoxazolyle, thiazolyle, oxazolyle, isoxazolyle ou isothiazolyle portant éventuellement un substituant alkyle en C₁ à C₆, alkoxy en C₁ à C₆, fluoro, chloro, bromo, trifluorométhyle, diméthylamino ou diéthylamino,
ou bien
R² et R³ forment conjointement avec l'atome d'azote un hétérocycle de la série 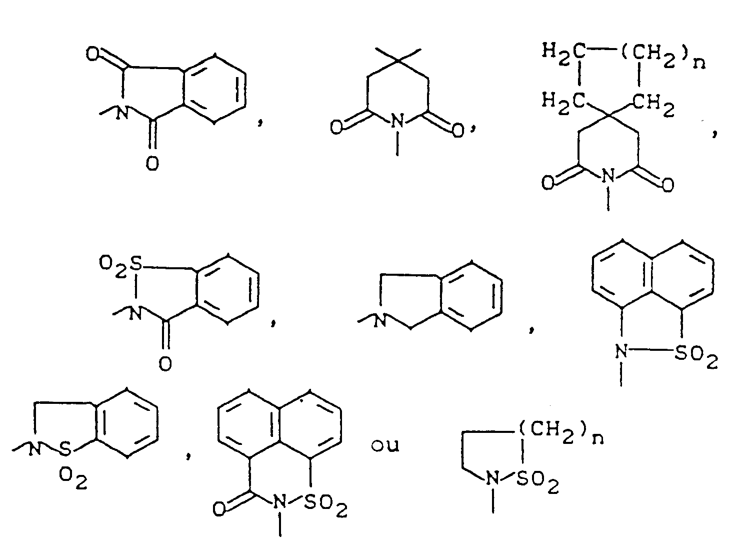 dans laquelle
n a la valeur 1 ou 2,
et leurs sels.

2. 1,3,4,5-tétrahydrobenz[c,d]indoles suivant la revendication 1,
dans lesquels
R¹ représente l'hydrogène, un groupe méthyle, éthyle, n-propyle ou benzyle,
X représente H, OCH₃, OH, SCH₃, F, Cl, CN, CONH₂,
Y désigne un groupe alkylène à chaîne droite ayant 2 à 4 atomes de carbone,
et
Z est un groupe cyano, ou un groupe de formule 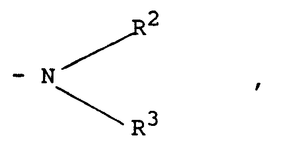 SO₂NR⁷R⁸, CONR⁷R⁸,
où
R⁷ et R⁸ sont identiques ou différents et représentent
l'hydrogène, un groupe méthyle, éthyle, propyle, isopropyle, butyle, isobutyle ou tertiobutyle,
R² et R³ sont identiques ou différents et représentent
l'hydrogène, un groupe méthyle, éthyle, propyle, isopropyle, butyle, isobutyle, tertiobutyle ou un groupe phényle éventuellement substitué par du fluor, du chlore, un radical méthyle ou méthoxy ou
un groupe -COR⁹ ou -SO₂R¹⁰,
où
R⁹ désigne l'hydrogène ou un groupe NHR¹¹, ou bien un groupe méthyle, éthyle, propyle, isopropyle, méthoxy, éthoxy, propoxy, isopropoxy, ou bien un groupe phényle, benzyle, benzyloxy, thiényle, furyle, pyridyle, pyrimidyle, quinolyle ou isoquinolyle portant éventuellement un substituant méthyle, méthoxy, fluoro ou chloro,
R¹⁰ est un groupe méthyle, éthyle, propyle, isopropyle, butyle ou isobutyle portant éventuellement un substituant fluoro, chloro, méthoxycarbonyle, éthoxycarbonyle, propoxycarbonyle, isopropoxycarbonyle, butoxycarbonyle ou isobutoxycarbonyle, ou bien un groupe phényle, naphtyle, benzyle, thiényle, furyle, pyridyle, pyrimidyle, quinolyle ou isoquinolyle portant facultativement un substituant méthyle, éthyle, propyle, isopropyle, méthoxy, fluoro ou chloro, ou bien
un groupe NR⁷R⁸,
dans lequel
R⁷ et R⁸ ont la définition indiquée ci-dessus,
et
R¹¹ est un groupe méthyle, éthyle, propyle, isopropyle, butyle, isobutyle, pentyle, isopentyle, hexyle ou isohexyle portant facultativement un substituant fluoro ou chloro, ou bien un groupe phényle ou benzyle qui peut être substitué par du fluor, du chlore, un radical méthyle ou méthoxy,
ou bien
R² et R³ forment conjointement avec l'atome d'azote un hétérocycle de la série 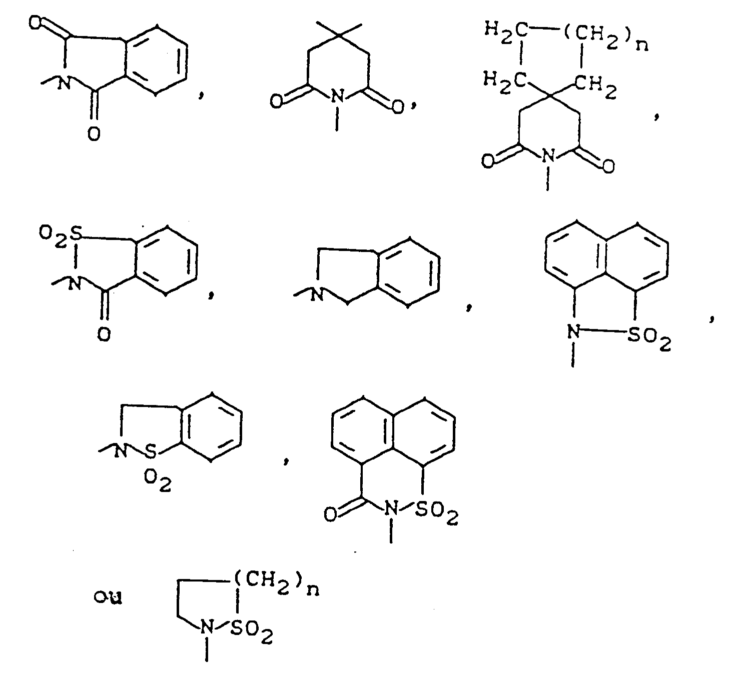 où
n a la valeur 1 ou 2
et leurs sels.

3. 1,3,4,5-tétrahydrobenz[c,d]indoles suivant la revendication 1, destiné au traitement thérapeutique.

4. Procédé de production de 1,3,4,5-tétrahydrobenz[c,d]indoles suivant la revendication 1, caractérisé en ce qu'on amine [A] par voie de réduction des cétones de formule (II) 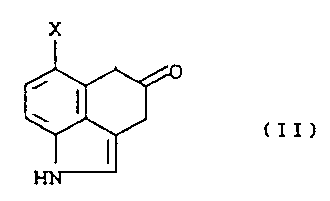 dans laquelle X a la définition indiquée dans la revendication 1
avec des amines de formule (III), (IV) ou (V) 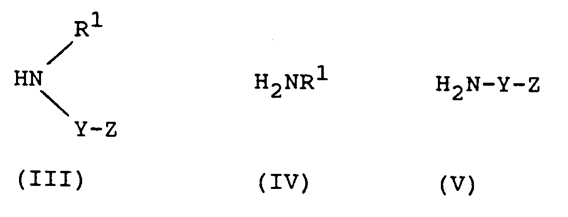 où R¹, Y et Z ont la définition indiquée dans la revendication 1.

5. Procédé de production de 1,3,4,5-tétrahydrobenz[c,d]indoles suivant la revendication 1,
caractérisé en ce qu'on fait réagir [B] des composés de formule générale (VI) 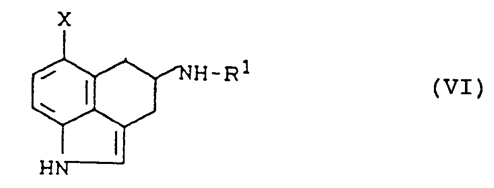 dans laquelle R¹ et X ont la définition indiquée dans la revendication 1,
avec des agents d'alkylation de formule (VII)
L-Y-Z (VII)
dans laquelle Y et Z ont la définition indiquée dans la revendication 1 et L est un "leaving group" pour agents d'alkylation,
ou bien on les alkyle [C] par voie de réduction avec des aldéhydes de formule (VIII)
OCH-Y¹-Z (VIII)
dans lesquels Z a la définition indiquée dans la revendication 1 et Y¹ est une chaîne alkylène Y raccourcie d'un groupe méthylène,
ou bien on les fait réagir avec des dérivés réactifs d'acides de formule générale (IX)
M-OC-Y¹-Z (IX)
dans laquelle Y¹ et Z ont la définition indiquée ci-dessus et M est un leaving group classique dans des agents d'acylation, tel que le chlore, le brome, un groupe alkoxy, aryloxy, imidazolyle, thiazolyle, méthanesulfonyloxy ou alkoxycarbonyloxy et on réduit [D] les amides d'acides obtenus, par voie catalytique avec du peroxyde d'hydrogène ou avec des hydrures métalliques complexes, en composés de formule (I).

6. Procédé de production de 1,3,4,5-tétrahydrobenz[c,d]indoles suivant la revendication 1, caractérisé en ce qu'on alkyle des composés de formule (X) 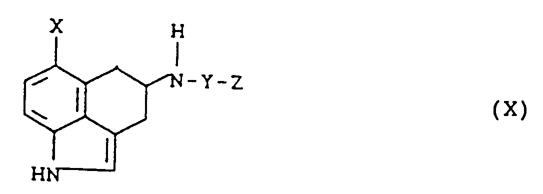 dans laquelle X, Y et Z ont la définition indiquée dans la revendication 1,
avec des agents d'alkylation de formule (XI)
R¹-L (XI)
dans laquelle R¹ et L ont la définition indiquée dans la revendication 1,
ou bien on les alkyle par voie de réduction avec des aldéhydes de formule (XII)
R¹³-CHO (XII)
dans laquelle R¹³ est un reste R¹ raccourci d'un groupe méthylène
ou bien on les fait réagir avec des dérivés d'acides réactifs de formule générale (XIII)
M-CO-R¹³ (XIII)
dans laquelle R¹³ a la définition indiquée ci-dessus et M a la définition indiquée dans la revendication 5 et on réduit les amides d'acides obtenus par voie catalytique avec du peroxyde d'hydrogène ou avec des hydrures métalliques complexes en composés de formule (I).

7. Médicament contenant des 1,3,4,5-tétrahydrobenz[c,d]indoles suivant la revendication 1.

8. Médicament suivant la revendication 7, caractérisé en ce qu'il contient 0,5 à 90 % en poids de 1,3,4,5-tétrahydrobenz[c,d]indoles, par rapport au mélange total.

9. Utilisation des 1,3,4,5-tétrahydrobenz[c,d]indoles suivant la revendication 1 pour la préparation de médicaments.

10. Utilisation suivant la revendication 9 pour la préparation de médicaments destinés au traitement de maladies du système nerveux central.

## Revendications (Revendications pour l'(les) Etat(s) contractant(s) suivant(s): ES)

1. Procédé de production de 1,3,4,5-tétrahydrobenz[c,d]indoles de formule 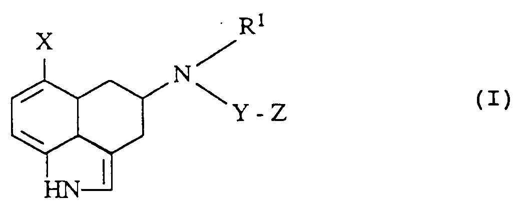 dans laquelle
R¹ représente H, un groupe alkyle en C₁ à C₄ ou benzyle,
X représente H, un groupe OCH₃, OH, SCH₃, F, Cl, Br, CN ou CONH₂,
Y est une chaîne alkylène droite ou ramifiée, saturée ou non saturée, ayant jusqu'à 6 atomes de carbone,
et
Z est un groupe cyano, ou un groupe de formule 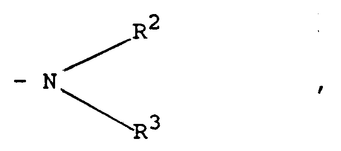 SO₂NR⁷R⁸ ou CONR⁷R⁸,
où
R⁷ et R⁸ sont identiques ou différents et représentent
l'hydrogène, un groupe alkyle en C₁ à C₆, phényle, benzyle ou phénéthyle
R² et R³ sont identiques ou différents et représentent
l'hydrogène ou un groupe alkyle en C₁ à C₆, phényle ou benzyle, les restes phényle pouvant être substitués par du fluor, du chlore, du brome, un groupe alkyle en C₁ à C₆, alkoxy en C₁ à C₆ ou trifluorométhyle, ou bien
un groupe de formule
COR⁹ ou -SO₂R¹⁰,
où
R⁹ est l'hydrogène ou un groupe NHR¹¹ ou bien un groupe alkyle en C₁ à C₆ ou alkoxy en C₁ à C₆, ou bien un groupe phényle, benzyle, benzyloxy, thiényle, furyle, pyridyle, pyrimidyle, quinolyle, isoquinolyle, benzothiazolyle, benzoxazolyle, thiazolyle, oxazolyle, isoxazolyle ou isothiazolyle portant éventuellement un substituant alkyle en C₁ à C₆, alkoxy en C₁ à C₆, fluoro, chloro, bromo, trifluorométhyle, diméthylamino ou diéthylamino,
R¹⁰ désigne un groupe alkyle en C₁ à C₆ portant facultativement un substituant cyano, fluoro, chloro, bromo, trifluorométhyle ou (alkoxy en C₁ à C₆)carbonyle, ou bien un groupe phényle, naphtyle, benzyle, thiényle, furyle, pyrimidyle, pyridyle, quinolyle, isoquinolyle, benzothiazolyle, benzoxazolyle, thiazolyle, oxazolyle, isoxazolyle ou isothiazolyle portant facultativement un substituant alkyle en C₁ à C₄, alkoxy en C₁ à C₄, fluoro, chloro, bromo, trifluorométhyle, diéthylamino ou diméthylamino, ou bien
un groupe -NR⁷R⁸,
dans lequel
R⁷ et R⁸ ont la définition indiquée ci-dessus et
R¹¹ représente un groupe alkyle en C₁ à C₆ portant éventuellement un substituant cyano, fluoro, chloro ou bromo, ou un groupe phényle, benzyle, thiényle, furyle, pyridyle, pyrimidyle, quinolyle, isoquinolyle, benzothiazolyle, benzoxazolyle, thiazolyle, oxazolyle, isoxazolyle ou isothiazolyle portant éventuellement un substituant alkyle en C₁ à C₆, alkoxy en C₁ à C₆, fluoro, chloro, bromo, trifluorométhyle, diméthylamino ou diéthylamino,
ou bien
R² et R³ forment conjointement avec l'atome d'azote un hétérocycle de la série 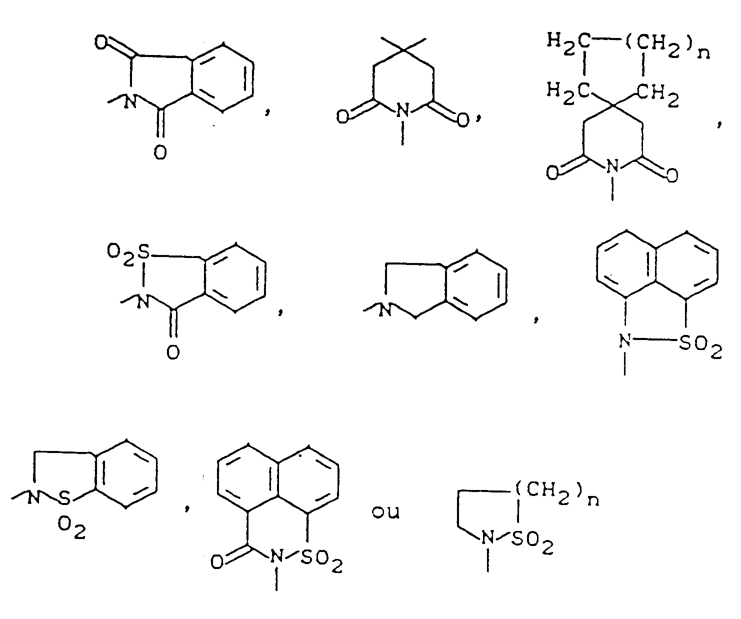 dans laquelle
n a la valeur 1 ou 2,
et de leurs sels,
caractérisé en ce qu'on amine [A] par voie de réduction des cétones de formule (II) 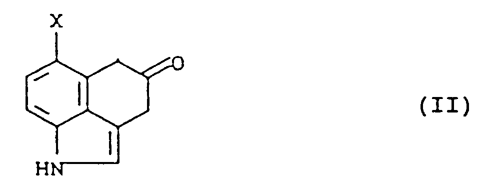 dans laquelle X a la définition indiquée ci-dessus,
avec des amines de formule (III), (IV) ou (V) 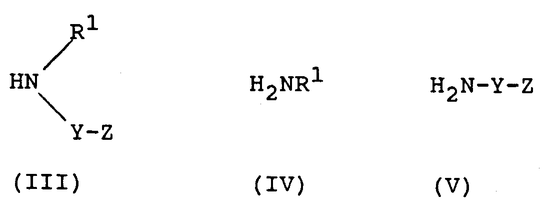 dans lesquelles R¹, Y et Z ont la définition indiquée ci-dessus.

2. Procédé de production de 1,3,4,5-tétrahydrobenz[c,d]indoles suivant la revendication 1, caractérisé en ce qu'on fait réagir [B] des composés de formule générale (VI) 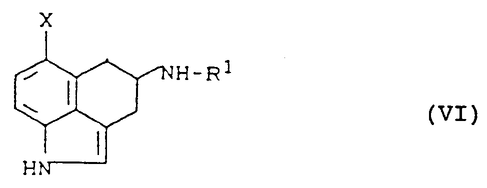 dans laquelle R¹ et X ont la définition indiquée dans la revendication 1,
avec des agents d'alkylation de formule (VII)
L-Y-Z (VII)
dans laquelle Y et Z ont la définition indiquée dans la revendication 1 et L est un "leaving group" pour agents d'alkylation,
ou bien on les alkyle [C] par voie de réduction avec des aldéhydes de formule (VIII)
OCH-Y¹-Z (VIII)
dans lesquels Z a la définition indiquée dans la revendication 1 et Y¹ est une chaîne alkylène Y raccourcie d'un groupe méthylène,
ou bien on les fait réagir avec des dérivés réactifs d'acides de formule générale (IX)
M-OC-Y¹-Z (IX)
dans laquelle Y¹ et Z ont la définition indiquée ci-dessus et M est un leaving group classique dans des agents d'acylation, tel que le chlore, le brome, un groupe alkoxy, aryloxy, imidazolyle, thiazolyle, méthanesulfonyloxy ou alkoxycarbonyloxy et on réduit [D] les amides d'acides obtenus, par voie catalytique avec du peroxyde d'hydrogène ou avec des hydrures métalliques complexes, en composés de formule (I).

3. Procédé de production de 1,3,4,5-tétrahydrobenz[c,d]indoles suivant la revendication 1, caractérisé en ce qu'on alkyle des composés de formule (X) 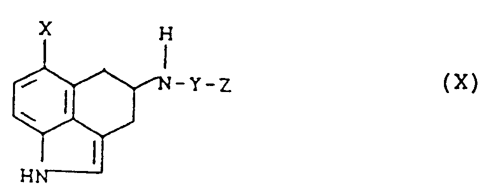 dans laquelle X, Y et Z ont la définition indiquée dans la revendication 1,
avec des agents d'alkylation de formule (XI)
R¹-L (XI)
dans laquelle R¹ et L ont la définition indiquée dans la revendication 1,
ou bien on les alkyle par voie de réduction avec des aldéhydes de formule (XII)
R¹³-CHO (XII)
dans laquelle R¹³ est un reste R¹ raccourci d'un groupe méthylène
ou bien on les fait réagir avec des dérivés d'acides réactifs de formule générale (XIII)
M-CO-R¹³ (XIII)
dans laquelle R¹³ a la définition indiquée ci-dessus et M a la définition indiquée dans la revendication 2 et on réduit les amides d'acides obtenus par voie catalytique avec du peroxyde d'hydrogène ou avec des hydrures métalliques complexes en composés de formule (I).

4. Procédé suivant la revendication 1, caractérisé en ce qu'on effectue l'amination par voie de réduction avec des borohydrures ou des hydrures d'aluminium complexes.
